# EUROPEAN PATENT SPECIFICATION

(11) **EP 4 079 306 B1**
(45) Date of publication and mention of the grant of the patent: **18.02.2026**
(21) Application number: 20903242.4
(22) Date of filing: 18.12.2020
(51) Int. Cl.: A61K 31/519, A61P 13/12, A61P 43/00, C07D 487/04

(54) **BTK INHIBITOR FOR USE IN THE TREATMENT OF IGA NEPHROPATHY OR PURPURA NEPHRITIS**
BTK HEMMER ZUR VERWENDUNG IN DER BEHANDLUNG VON IGA NEPHRITIS ODER PURPURA NEPHRITIS
INHIBITEUR DE LA BTK POUR L'UTILISATION DANS LE TRAITEMENT DE LA NÉPHROPATHIE À IGA OU DE LA NÉPHROPATHIE DU PURPURA RHUMATOÏDE

(30) Priority: 19.12.2019 JP 2019229171
(43) Date of publication of application: 26.10.2022
(73) Proprietor: Taiho Pharmaceutical Co., Ltd., Chiyoda-ku Tokyo 101-8444 (JP)
(72) Inventor: ARIMA Yasunobu, Tsukuba-shi, Ibaraki 300-2611 (JP); KANEKO Ryusuke, Tsukuba-shi, Ibaraki 300-2611 (JP)
(74) Representative: Wächtershäuser & Hartz Patentanwaltspartnerschaft mbB
(86) International application number: PCT/JP2020/047370
(87) International publication number: WO 2021/125313

(56) References cited:
- EP-A1- 3 115 365
- EP-A1- 3 120 852
- WO-A1-2015/022926
- WO-A1-2015/170266
- WO-A1-2016/121953
- WO-A1-2016/121954
- WO-A1-2016/187723
- WO-A1-2019/043609
- JP-A- 2017 533 881
- CHEN JIN-SHUEN, CHANG LI-CHIEN, HUANG SHYH-JER, CHENG CHAO-WEN: "Targeting Spleen Tyrosine Kinase-Bruton’s Tyrosine Kinase Axis for Immunologically Mediated Glomerulonephritis", BIOMED RESEARCH INTERNATIONAL, HINDAWI PUBLISHING CORPORATION, vol. 2014, 1 January 2014 (2014-01-01), pages 1 - 6, XP055836051, ISSN: 2314-6133, DOI: 10.1155/2014/814869
- CHALMERS SAMANTHA A., CHALMERS SAMANTHA, DOERNER JESSICA, BOSANAC TODD, KHALIL SARA, SMITH DUSTIN, HARCKEN CHRISTIAN, DIMOCK JANIC: "Therapeutic Blockade of Immune Complex-Mediated Glomerulonephritis by Highly Selective Inhibition of Bruton’s Tyrosine Kinase", SCIENTIFIC REPORTS, NATURE PUBLISHING GROUP, vol. 6, no. 1, 1 May 2016 (2016-05-01), pages 26164, XP055836052, DOI: 10.1038/srep26164

## Description

### Technical Field

The present invention relates to a therapeutic agent for use in the treatment of IgA nephropathy, or purpura nephritis, wherein the therapeutic agent is (R) -1- (1-acryloylpiperidin-3-yl)-4-amino-N-(benzo[d]oxazol-2-yl)-1H-pyrazolo[3,4-d]pyrimidine-3-carboxamide (hereafter referred to as Compound 1) of having a Bruton's tyrosine kinase (BTK) inhibitory action, or a salt thereof.

### Background Art

Glomerular disease (primary glomerulonephritis) involving a lesion in the glomerulus of the kidney is classified into 7 diseases, namely, IgA nephropathy, membranous nephropathy, membranoproliferative glomerulonephritis, acute poststreptococcal glomerulonephritis, minimal change nephrotic syndrome (lipoid nephrosis), focal glomerulosclerosis, and rapidly progressive glomerulonephritis. Among these diseases, IgA nephropathy, membranous nephropathy, membranoproliferative glomerulonephritis, purpura nephritis, and focal glomerulosclerosis are generally referred to as "chronic glomerulonephritis." The cause of the onset of chronic glomerulonephritis and the onset time thereof are relatively unclear in many cases, and further, it is considered that the damage process is progressive and finally reaches renal failure in many cases.

In the case of chronic glomerulonephritis, a lesion is frequently observed in the glomerulus, and it is considered that the formation of such a glomerular lesion is associated with proliferation of mesangial cells as one type of glomerulus-constituting cells, accumulation of the extracellular matrix thereof (mesangial matrix), and thickening of the basement membrane.

Among others, IgA nephropathy is the most common chronic glomerulonephritis. IgA nephropathy is a disease, in which a deposit mainly comprising IgA is observed in a renal glomerular mesangial region, and proliferation of mesangial cells and an increase and expansion of the mesangial matrix occurs. The percentage of IgA nephropathy in all chronic glomerulonephritis diseases is 10% in North America, 20% in Europe, and 30% to 40% in the Asia-Pacific region involving regional uneven distribution (Non-Patent Literature 1). The gender ratio is 2 : 1 to 6 : 1 (male : female), and it seems that IgA nephropathy is preferentially developed in male patients. Approximately 40% of IgA nephropathy patients reach terminal-stage renal failure, and thus, this is a poor prognostic disease (Non-Patent Literature 2).

Regarding IgA nephropathy, it has been known that IgG specific to galactose-deficient sugar chain modified IgA is produced, and that such IgG and IgA form a polymer immune complex, which is deeply associated with IgA nephropathy. The formed polymer immune complex is deposited in the mesangial region, and the impairment of the glomerulus is induced by activation of mesangial cells and production of inflammatory cytokines, thereby resulting in a decrease in renal function (Non-Patent Literatures 3 and 4). In addition, it has been known that IgG that forms a polymer immune complex induces production of inflammatory cytokines via FCγ receptors, and that this induction is deeply associated with the onset of the disease (Non-Patent Literature 5).

At present, a fundamental treatment method has not been established as a treatment of IgA nephropathy, and thus, a symptomatic therapy has been performed on IgA nephropathy. Specifically, IgA nephropathy is treated using a renin-angiotensin inhibitor, an adrenocorticosteroid drug, an immunosuppressant, palate tonsillectomy, an antiplatelet drug, or n-3 fatty acid. In addition, depending on the cases, blood pressure control, salt reduction, lipid control, blood sugar control, weight management, and smoking cessation are carried out, but a fundamental treatment method has not yet been established (Non-Patent Literature 6).

As such, intensive studies have been conducted to elucidate a mechanism of the onset of chronic glomerulonephritis including IgA nephropathy as a typical example or to develop a treatment method for chronic glomerulonephritis. However, a therapeutic agent has not yet been developed, and thus, it has been strongly desired to develop a novel therapeutic agent for chronic glomerulonephritis. In particular, there are no approved drugs for IgA nephropathy at the present moment. Approximately 40% of patients with IgA nephropathy reach renal failure, and are affected with complications such as hypertension and electrolyte abnormality. Hence, it has been desired to create a novel therapeutic agent (Non-Patent Literature 7).

It has been known that a protein kinase is present in various sites in a living body and is associated with a wide range of functional regulation. Bruton's tyrosine kinase (BTK) is a protein kinase belonging to a Tec kinase family, and BTK is expressed in myeloid cells such as B cells, monocytes/macrophages, neutrophils, mast cells and osteoclasts, and is associated with regulation of the functions of these cells (Non-Patent Literatures 8 and 9). BTK is located downstream of immune receptor signals such as B cell receptor (BCR) or Fc receptors (FcR) family. In the case of B cells, BTK is associated with proliferation, survival, differentiation and activation of the cells, whereas in the case of monocytes/macrophages, mast cells and the like, BTK is associated with regulation of the expression of inflammatory cytokines (tumor necrosis factor alpha (TNFα), interleukin 1β (IL-1β), etc.) or chemical transmitters (histamine, leukotriene, etc.) (Non-Patent Literature 10). It is considered that an inhibitor capable of regulating the activity of BTK will be useful as a therapeutic agent for diseases associated with abnormal acceleration of a BTK signal pathway (for example, cancer, allergic disease, and autoimmune disease).

In recent years, it has been considered that, in addition to B cells that are associated with antibody production, various cells such as monocytes/macrophages, neutrophils, mast cells and osteoclasts, which express Fc receptors (FcR) or related molecules thereof, are deeply associated with the onset and progression of autoimmune diseases such as rheumatoid arthritis (Non-Patent Literature 11). Since activation of these cells or abnormal acceleration of the functions thereof is associated with BTK signals (Non-Patent Literatures 9 and 10), a compound having a BTK inhibitory activity is expected to exhibit therapeutic effects on autoimmune diseases. Moreover, since BTK is also associated with activation of mast cells, a compound having a BTK inhibitory activity is expected to exhibit therapeutic effects on allergic diseases associated with B cells or mast cells.

It has been known that a fused pyrimidine compound group represented by a general formula (I) as shown below, or a salt thereof, has a high inhibitory activity against BTK and is useful as a therapeutic agent for cancer or autoimmune disease (Patent Literatures 1, 2, and 3). However, it has been totally unknown that this compound is effective for chronic glomerulonephritis such as IgA nephropathy.

The currently known BTK inhibitors include Ibrutinib (PCI-32765) and Acalabrutinib (ACP-196) (Non-Patent Literature 12). At present, in Japan, Ibrutinib has been approved with the name "Imbruvica" (registered trademark) as a drug for indications that are chronic lymphocytic leukemia and mantle cell lymphoma.

However, to date, the therapeutic effects of such BTK inhibitors on glomerulonephritis such as IgA nephropathy have not been known, and a treatment method therefor has not been established, either. JP 2017-533881 discloses the use of ginsenoside M1 for the treatment of IgA nephropathy but not any BTK inhibitor.

### Citation List

### Patent Literature

Patent Literature 1: International Publication WO2015/022926
Patent Literature 2: International Publication WO2016/121953
Patent Literature 3: International Publication WO2016/121954

### Non-Patent Literature

Non-Patent Literature 1: Prakash et al., Clin Nephrol. 2008 Nov; 70(5): 377-84.
Non-Patent Literature 2: Lai et al., Nat Rev Dis Primers. 2016 Feb 11; 2: 16001
Non-Patent Literature 3: Yeo et al., Pediatr Nephrol. 2018 May; 33(5): 763-777
Non-Patent Literature 4: Suzuki et al., Clinical and Experimental Nephrology 2019 23: 26-31
Non-Patent Literature 5: Lim et al., Nephrology 2003 8(1): 21-7)
Non-Patent Literature 6: Lai et al., Kidney Dis 2015 1(1): 19-26.
Non-Patent Literature 7: Donadio et al., N Engl J Med 2002; 347: 738-748
Non-Patent Literature 8: Schaeffer and Schwartzberg, Curr Op Imm, 2000, pp. 282-288
Non-Patent Literature 9: Schmidt U., et al., Int Arch Allergy Immunol, 134, 2004
Non-Patent Literature 10: Ellmeier W., et al., FEBS Journal., 278, 2011
Non-Patent Literature 11: Rommel C., et al., Nature reviews immunology, 7, 2007
Non-Patent Literature 12: Chengyuan L., et al., Eur J Med 151, 2018; 315-326

### Summary of Invention

### Technical Problem

Under such circumstances, it is strongly desired to develop a novel therapeutic agent for chronic glomerulonephritis.

### Solution to Problem

As a result of intensive studies directed towards achieving the aforementioned object, the present inventors have found that a fused pyrimidine compound group having a BTK inhibitory action or a pharmaceutically acceptable salt thereof is useful as a therapeutic agent for chronic glomerulonephritis, thereby completing the present invention.

Specifically, the present disclosure includes the following aspects.
[1-1] A therapeutic agent for chronic glomerulonephritis, comprising, as an active ingredient, a compound represented by the following general formula (I) or a salt thereof: 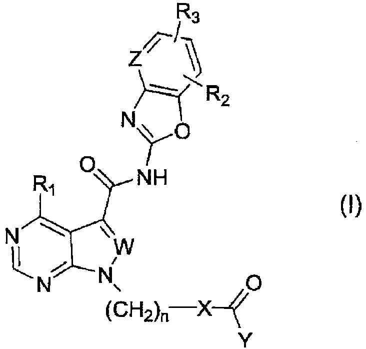
   wherein X represents nitrogen-containing C3-C10 heterocycloalkylene optionally having a substituent;
   Y represents -C(R₄)=C(R₅)(R₆) or -C≡C-R₇;
   W and Z each independently represent N or CH;
   n represents an integer of 0 to 2;
   R₁ represents an amino group optionally having a substituent;
   R₂ and R₃, which are the same or different, each represent a hydrogen atom, a halogen atom, a C1-C6 alkyl group optionally having a substituent, a C1-C6 alkoxy group optionally having a substituent, a C3-C7 cycloalkyl group optionally having a substituent, a C6-C14 aromatic hydrocarbon group optionally having a substituent, a 4- to 10-membered monocyclic or polycyclic unsaturated heterocyclic group optionally having a substituent and containing 1 to 3 heteroatoms of the same or different types selected from a nitrogen atom, an oxygen atom and a sulfur atom, or a cyano group; and
   R₄, R₅, R₆ and R₇, which are the same or different, each represent a hydrogen atom or a C1-C6 alkyl group optionally having a substituent.
[1-2] The therapeutic agent according to the above [1-1], which has an IgG production inhibitory action.
[1-3] The therapeutic agent according to the above [1-1] or [1-2], which has an action to inhibit the production of inflammatory cytokines mediated by an immune complex-FCy receptor.
[2] The therapeutic agent according to any one of the above [1-1] to [1-3], wherein, in the general formula (I),
   X is 1,3-piperidinylene;
   Y is a vinyl group;
   Z is CH;
   W is N;
   n is 0;
   R₁ is an amino group; and
   either one of R₂ and R₃ is a hydrogen atom, and the other is a hydrogen atom, a halogen atom, or a cyano group.
[3] The therapeutic agent according to [1-1] to [1-3] or [2], the active ingredient of which is (R)-1-(1-acryloylpiperidin-3-yl)-4-amino-N-(benzo[d]oxazol-2-yl)-1H-pyrazolo[3,4-d]pyrimidine-3-carboxamide or a salt thereof.
[4-1] The therapeutic agent according to any one of the above [1-1] to [1-3], [2], and [3], wherein the chronic glomerulonephritis is IgA nephropathy or purpura nephritis.
[4-2] The therapeutic agent according to any one of the above [1-1] to [1-3], [2], [3], and [4-1], wherein the chronic glomerulonephritis is IgA nephropathy.
[4-3] The therapeutic agent according to any one of the above [1-1] to [1-3], [2], [3], [4-1], and [4-2], which is for use in oral administration.
[4-4] The therapeutic agent according to any one of the above [1-1] to [1-3], [2], [3], and [4-1] to [4-3], which is used in combination with another therapeutic agent or a surgical treatment.
[4-5] The therapeutic agent according to any one of the above [1-1] to [1-3], [2], [3], and [4-1] to [4-4], which is used in combination with tonsillectomy pulse therapy.
[5-1] A pharmaceutical composition for treating chronic glomerulonephritis, which comprises, as an active ingredient, a compound represented by the following general formula (I) or a salt thereof: 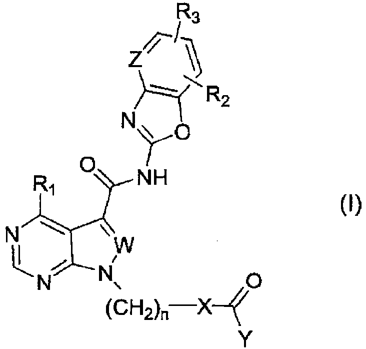
   wherein X represents nitrogen-containing C3-C10 heterocycloalkylene optionally having a substituent;
   Y represents -C(R₄)=C(R₅)(R₆) or -C≡C-R₇;
   W and Z each independently represent N or CH;
   n represents an integer of 0 to 2;
   R₁ represents an amino group optionally having a substituent;
   R₂ and R₃, which are the same or different, each represent a hydrogen atom, a halogen atom, a C1-C6 alkyl group optionally having a substituent, a C1-C6 alkoxy group optionally having a substituent, a C3-C7 cycloalkyl group optionally having a substituent, a C6-C14 aromatic hydrocarbon group optionally having a substituent, a 4- to 10-membered monocyclic or polycyclic unsaturated heterocyclic group optionally having a substituent and containing 1 to 3 heteroatoms of the same or different types selected from a nitrogen atom, an oxygen atom and a sulfur atom, or a cyano group; and
   R₄, R₅, R₆ and R₇, which are the same or different, each represent a hydrogen atom or a C1-C6 alkyl group optionally having a substituent.
[5-2] The pharmaceutical composition according to the above [5-1], which has an IgG production inhibitory action.
[5-3] The pharmaceutical composition according to the above [5-1] or [5-2], which has an action to inhibit the production of inflammatory cytokines mediated by an immune complex-FCy receptor.
[6] The pharmaceutical composition according to any one of the above [5-1] to [5-3], wherein, in the general formula (I),
   X is 1,3-piperidinylene;
   Y is a vinyl group;
   Z is CH;
   W is N;
   n is 0;
   R₁ is an amino group; and
   either one of R₂ and R₃ is a hydrogen atom, and the other is a hydrogen atom, a halogen atom, or a cyano group.
[7] The pharmaceutical composition according to any one of the above [5-1] to [5-3] and [6], the active ingredient of which is (R)-1-(1-acryloylpiperidin-3-yl)-4-amino-N-(benzo[d]oxazol-2-yl)-1H-pyrazolo[3,4-d]pyrimidine-3-carboxamide or a salt thereof.
[8-1] The pharmaceutical composition according to any one of the above [5-1] to [5-3], [6], and [7], wherein the chronic glomerulonephritis is IgA nephropathy or purpura nephritis.
[8-2] The pharmaceutical composition according to any one of the above [5-1] to [5-3], [6], [7], and [8-1], wherein the chronic glomerulonephritis is IgA nephropathy.
[8-3] The pharmaceutical composition according to any one of the above [5-1] to [5-3], [6], [7], [8-1], and [8-2], which is for use in oral administration.
[8-4] The pharmaceutical composition according to any one of the above [5-1] to [5-3], [6], [7], and [8-1] to [8-3], which is used in combination with another therapeutic agent or a surgical treatment.
[8-5] The pharmaceutical composition according to any one of the above [5-1] to [5-3], [6], [7], and [8-1] to [8-4], which is used in combination with tonsillectomy pulse therapy.
[9-1] A method for treating chronic glomerulonephritis, comprising a step of administering an effective amount of a compound represented by the following general formula (I) or a salt thereof to a subject in need thereof: 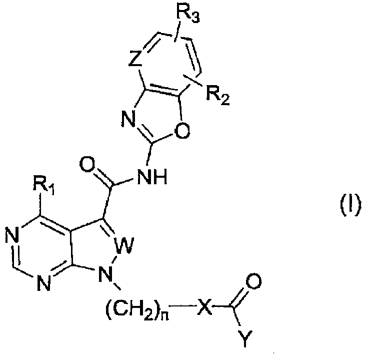
   wherein X represents nitrogen-containing C3-C10 heterocycloalkylene optionally having a substituent;
   Y represents -C(R₄)=C(R₅)(R₆) or -C≡C-R₇;
   W and Z each independently represent N or CH;
   n represents an integer of 0 to 2;
   R₁ represents an amino group optionally having a substituent;
   R₂ and R₃, which are the same or different, each represent a hydrogen atom, a halogen atom, a C1-C6 alkyl group optionally having a substituent, a C1-C6 alkoxy group optionally having a substituent, a C3-C7 cycloalkyl group optionally having a substituent, a C6-C14 aromatic hydrocarbon group optionally having a substituent, a 4- to 10-membered monocyclic or polycyclic unsaturated heterocyclic group optionally having a substituent and containing 1 to 3 heteroatoms of the same or different types selected from a nitrogen atom, an oxygen atom and a sulfur atom, or a cyano group; and
   R₄, R₅, R₆ and R₇, which are the same or different, each represent a hydrogen atom or a C1-C6 alkyl group optionally having a substituent.
[9-2] The method according to the above [9-1], wherein IgG production is inhibited by the administration.
[9-3] The method according to the above [9-1] or [9-2], wherein the production of inflammatory cytokines mediated by an immune complex-FCy receptor is inhibited by the administration.
[10] The method according to any one of the above [9-1] to [9-3], wherein, in the general formula (I),
   X is 1,3-piperidinylene;
   Y is a vinyl group;
   Z is CH;
   W is N;
   n is 0;
   R₁ is an amino group; and
   either one of R₂ and R₃ is a hydrogen atom, and the other is a hydrogen atom, a halogen atom, or a cyano group.
[11] The method according to [9-1] to [9-3] or [10], wherein the compound represented by the general formula (I) is (R)-1-(1-acryloylpiperidin-3-yl)-4-amino-N-(benzo[d]oxazol-2-yl)-1H-pyrazolo[3,4-d]pyrimidine-3-carboxamide or a salt thereof.
[12-1] The method according to any one of the above [9-1] to [9-3], [10], and [11], wherein the chronic glomerulonephritis is IgA nephropathy or purpura nephritis.
[12-2] The method according to any one of the above [9-1] to [9-3], [10], [11], and [12-1], wherein the chronic glomerulonephritis is IgA nephropathy.
[12-3] The method according to any one of the above [9-1] to [9-3], [10], [11], [12-1], and [12-2], wherein the compound represented by the general formula (I) or a salt thereof is orally administered.
[12-4] The method according to any one of the above [9-1] to [9-3], [10], [11], and [12-1] to [12-3], which is used in combination with another therapeutic agent or a surgical treatment.
[12-5] The method according to any one of the above [9-1] to [9-3], [10], [11], and [12-1] to [12-4], which is used in combination with tonsillectomy pulse therapy.
[13-1] A compound represented by the following general formula (I) or a salt thereof, for use in the treatment of chronic glomerulonephritis: 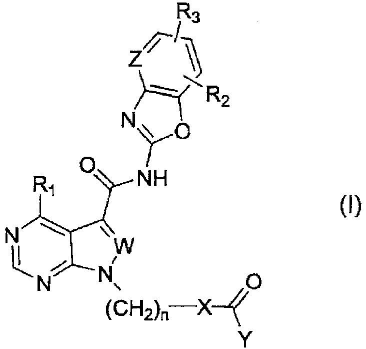
   wherein X represents nitrogen-containing C3-C10 heterocycloalkylene optionally having a substituent;
   Y represents -C(R₄)=C(R₅)(R₆) or -C≡C-R₇;
   W and Z each independently represent N or CH;
   n represents an integer of 0 to 2;
   R₁ represents an amino group optionally having a substituent;
   R₂ and R₃, which are the same or different, each represent a hydrogen atom, a halogen atom, a C1-C6 alkyl group optionally having a substituent, a C1-C6 alkoxy group optionally having a substituent, a C3-C7 cycloalkyl group optionally having a substituent, a C6-C14 aromatic hydrocarbon group optionally having a substituent, a 4- to 10-membered monocyclic or polycyclic unsaturated heterocyclic group optionally having a substituent and containing 1 to 3 heteroatoms of the same or different types selected from a nitrogen atom, an oxygen atom and a sulfur atom, or a cyano group; and
   R₄, R₅, R₆ and R₇, which are the same or different, each represent a hydrogen atom or a C1-C6 alkyl group optionally having a substituent.
[13-2] The compound according to the above [13-1] or a salt thereof, which has an IgG production inhibitory action.
[13-3] The compound according to the above [13-1] or [13-2], or a salt thereof, which has an action to inhibit the production of inflammatory cytokines mediated by an immune complex-FCy receptor.
[14] The compound according to any one of the above [13-1] to [13-3], or a salt thereof, wherein, in the general formula (I),
   X is 1,3-piperidinylene;
   Y is a vinyl group;
   Z is CH;
   W is N;
   n is 0;
   R₁ is an amino group; and
   either one of R₂ and R₃ is a hydrogen atom, and the other is a hydrogen atom, a halogen atom, or a cyano group.
[15] The compound according to any one of the above [13-1] to [13-3] and [14], or a salt thereof, the active ingredient of which is (R)-1-(1-acryloylpiperidin-3-yl)-4-amino-N-(benzo[d]oxazol-2-yl)-1H-pyrazolo[3,4-d]pyrimidine-3-carboxamide or a salt thereof.
[16-1] The compound according to any one of the above [13-1] to [13-3], [14], and [15], or a salt thereof, wherein the chronic glomerulonephritis is IgA nephropathy or purpura nephritis.
[16-2] The compound according to any one of the above [13-1] to [13-3], [14], [15], and [16-1], or a salt thereof, wherein the chronic glomerulonephritis is IgA nephropathy.
[16-3] The compound according to any one of the above [13-1] to [13-3], [14], [15], [16-1], and [16-2], or a salt thereof, which is orally administered to treat chronic glomerulonephritis.
[16-4] The compound according to any one of the above [13-1] to [13-3], [14], [15], and [16-1] to [16-3], or a salt thereof, which is used in combination with another therapeutic agent or a surgical treatment to treat chronic glomerulonephritis.
[16-5] The compound according to any one of the above [13-1] to [13-3], [14], [15], and [16-1] to [16-3], or a salt thereof, which is used in combination with tonsillectomy pulse therapy to treat chronic glomerulonephritis.
[17-1] Use of a compound represented by the following general formula (I) or a salt thereof for producing a therapeutic agent for chronic glomerulonephritis: 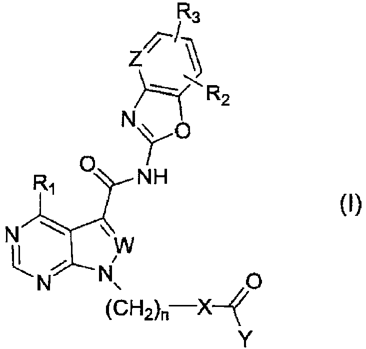
   wherein X represents nitrogen-containing C3-C10 heterocycloalkylene optionally having a substituent;
   Y represents -C(R₄)=C(R₅)(R₆) or -C≡C-R₇;
   W and Z each independently represent N or CH;
   n represents an integer of 0 to 2;
   R₁ represents an amino group optionally having a substituent;
   R₂ and R₃, which are the same or different, each represent a hydrogen atom, a halogen atom, a C1-C6 alkyl group optionally having a substituent, a C1-C6 alkoxy group optionally having a substituent, a C3-C7 cycloalkyl group optionally having a substituent, a C6-C14 aromatic hydrocarbon group optionally having a substituent, a 4- to 10-membered monocyclic or polycyclic unsaturated heterocyclic group optionally having a substituent and containing 1 to 3 heteroatoms of the same or different types selected from a nitrogen atom, an oxygen atom and a sulfur atom, or a cyano group; and
   R₄, R₅, R₆ and R₇, which are the same or different, each represent a hydrogen atom or a C1-C6 alkyl group optionally having a substituent.
[17-2] The use according to the above [17-1], wherein the therapeutic agent has an IgG production inhibitory action.
[17-3] The use according to the above [17-1] or [17-2], wherein the therapeutic agent has an action to inhibit the production of inflammatory cytokines mediated by an immune complex-FCy receptor.
[18] The use according to any one of the above [17-1] to [17-3], wherein, in the general formula (I),
   X is 1,3-piperidinylene;
   Y is a vinyl group;
   Z is CH;
   W is N;
   n is 0;
   R₁ is an amino group; and
   either one of R₂ and R₃ is a hydrogen atom, and the other is a hydrogen atom, a halogen atom, or a cyano group.
[19] The use according to any one of the above [17-1] to [17-3] and [18], wherein the compound represented by the general formula (I) is (R)-1-(1-acryloylpiperidin-3-yl)-4-amino-N-(benzo[d]oxazol-2-yl)-1H-pyrazolo[3,4-d]pyrimidine-3-carboxamide or a salt thereof.
[20-1] The use according to any one of the above [17-1] to [17-3], [18], and [19], wherein the chronic glomerulonephritis is IgA nephropathy or purpura nephritis.
[20-2] The use according to any one of the above [17-1] to [17-3], [18], [19], and [20-1], wherein the chronic glomerulonephritis is IgA nephropathy.
[20-3] The use according to any one of the above [17-1] to [17-3], [18], [19], [20-1], and [20-2], which is for producing a therapeutic agent for chronic glomerulonephritis that is for use in oral administration.
[20-4] The use according to any one of the above [17-1] to [17-3], [18], [19], and [20-1] to [20-3], which is for producing a therapeutic agent for chronic glomerulonephritis that is used in combination with another therapeutic agent or a surgical treatment.
[20-5] The use according to any one of the above [17-1] to [17-3], [18], [19], and [20-1] to [20-4], which is producing a therapeutic agent for chronic glomerulonephritis that is used in combination with tonsillectomy pulse therapy.
[21-1] A method for treating chronic glomerulonephritis, comprising administering a compound represented by the following general formula (I) or a salt thereof to a subject in need thereof, wherein the production of inflammatory cytokines by IgG that forms a polymer immune complex, mediated by an immune complex-FCy receptor, is inhibited by the administration: 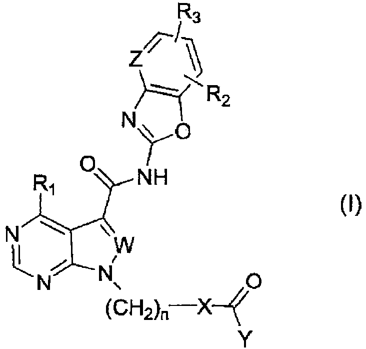
   wherein X represents nitrogen-containing C3-C10 heterocycloalkylene optionally having a substituent;
   Y represents -C(R₄)=C(R₅)(R₆) or -C≡C-R₇;
   W and Z each independently represent N or CH;
   n represents an integer of 0 to 2;
   R₁ represents an amino group optionally having a substituent;
   R₂ and R₃, which are the same or different, each represent a hydrogen atom, a halogen atom, a C1-C6 alkyl group optionally having a substituent, a C1-C6 alkoxy group optionally having a substituent, a C3-C7 cycloalkyl group optionally having a substituent, a C6-C14 aromatic hydrocarbon group optionally having a substituent, a 4- to 10-membered monocyclic or polycyclic unsaturated heterocyclic group optionally having a substituent and containing 1 to 3 heteroatoms of the same or different types selected from a nitrogen atom, an oxygen atom and a sulfur atom, or a cyano group; and
   R₄, R₅, R₆ and R₇, which are the same or different, each represent a hydrogen atom or a C1-C6 alkyl group optionally having a substituent.
[21-2] The method according to the above [21-1], wherein IgG production is inhibited by the administration.
[22] The method according to the above [21-1] or [21-2], wherein, in the general formula (I),
   X is 1,3-piperidinylene;
   Y is a vinyl group;
   Z is CH;
   W is N;
   n is 0;
   R₁ is an amino group; and
   either one of R₂ and R₃ is a hydrogen atom, and the other is a hydrogen atom, a halogen atom, or a cyano group.
[23] The method according to any one of the above [21-1], [21-2], and [22], wherein the compound represented by the general formula (I) is (R)-1-(1-acryloylpiperidin-3-yl)-4-amino-N-(benzo[d]oxazol-2-yl)-1H-pyrazolo[3,4-d]pyrimidine-3-carboxamide or a salt thereof.
[24-1] The method according to any one of the above [21-1], [21-2], [22], and [23], wherein the chronic glomerulonephritis is IgA nephropathy or purpura nephritis.
[24-2] The method according to any one of the above [21-1], [21-2], [22], [23], and [24-1], wherein the chronic glomerulonephritis is IgA nephropathy.
[24-3] The method according to any one of the above [21-1], [21-2], [22], [23], [24-1], and [24-2], wherein the compound represented by the general formula (I) or a salt thereof is orally administered.
[24-4] The method according to any one of the above [21-1], [21-2], [22], [23], and [24-1] to [24-3], which is used in combination with another therapeutic agent or a surgical treatment.
[24-5] The method according to any one of the above [21-1], [21-2], [22], [23], and [24-1] to [24-4], which is used in combination with tonsillectomy pulse therapy.
[25] A method for inhibiting a decrease in renal function, comprising administering a compound represented by the following general formula (I) or a salt thereof to a subject in need thereof: 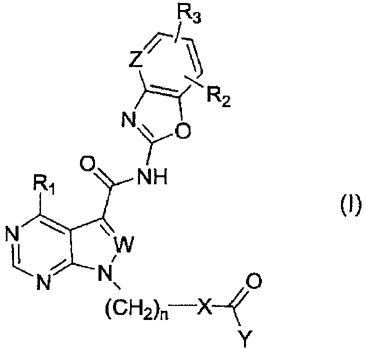
   wherein X represents nitrogen-containing C3-C10 heterocycloalkylene optionally having a substituent;
   Y represents -C(R₄)=C(R₅)(R₆) or -C≡C-R₇;
   W and Z each independently represent N or CH;
   n represents an integer of 0 to 2;
   R₁ represents an amino group optionally having a substituent;
   R₂ and R₃, which are the same or different, each represent a hydrogen atom, a halogen atom, a C1-C6 alkyl group optionally having a substituent, a C1-C6 alkoxy group optionally having a substituent, a C3-C7 cycloalkyl group optionally having a substituent, a C6-C14 aromatic hydrocarbon group optionally having a substituent, a 4- to 10-membered monocyclic or polycyclic unsaturated heterocyclic group optionally having a substituent and containing 1 to 3 heteroatoms of the same or different types selected from a nitrogen atom, an oxygen atom and a sulfur atom, or a cyano group; and
   R₄, R₅, R₆ and R₇, which are the same or different, each represent a hydrogen atom or a C1-C6 alkyl group optionally having a substituent.
[26] The method according to the above [25], wherein, in the general formula (I),
   X is 1,3-piperidinylene;
   Y is a vinyl group;
   Z is CH;
   W is N;
   n is 0;
   R₁ is an amino group; and
   either one of R₂ and R₃ is a hydrogen atom, and the other is a hydrogen atom, a halogen atom, or a cyano group.
[27] The method according to the above [25] or [26], wherein the compound represented by the general formula (I) is (R)-1-(1-acryloylpiperidin-3-yl)-4-amino-N-(benzo[d]oxazol-2-yl)-1H-pyrazolo[3,4-d]pyrimidine-3-carboxamide or a salt thereof.
[28] The method according to any one of the above [25] to [27], wherein the decrease in renal function is associated with chronic glomerulonephritis.
[29] The method according to the above [28], wherein the chronic glomerulonephritis is IgA nephropathy or purpura nephritis.
[30] The method according to the above [28] or [29], wherein the chronic glomerulonephritis is IgA nephropathy.
[31-1] The method according to any one of the above [25] to [30], wherein the compound represented by the general formula (I) or a salt thereof is orally administered.
[31-2] The method according to any one of the above [25] to [30] and [31-1], which is used in combination with another therapeutic agent or a surgical treatment.
[31-3] The method according to any one of the above [25] to [30], [31-1], and [31-2], which is used in combination with tonsillectomy pulse therapy.

### Advantageous Effects of Invention

According to the present invention, a novel therapeutic agent for IgA nephropathy or purpura nephritis is provided, which is (R)-1-(1-acryloylpiperidin-3-yl)-4-amino-N-(benzo[d]oxazol-2-yl)-1H-pyrazolo[3,4-d]pyrimidine-3-carboxamide (Compound 1).

### Brief Description of Drawings

[Figure 1] Figure 1 shows inhibition of IgG production in mouse B cells by Compound 1.
[Figure 2] Figure 2 shows inhibition of TNFα production in human monocytic cells by Compound 1.
[Figure 3] Figure 3 shows the effect of Compound 1 to inhibit a decrease in renal function in HIGA mice.

### Description of Embodiments

One aspect of the present disclosure relates to a therapeutic agent for chronic glomerulonephritis (for example, IgA nephropathy), comprising, as an active ingredient, a compound represented by the following general formula (I) or a salt thereof:

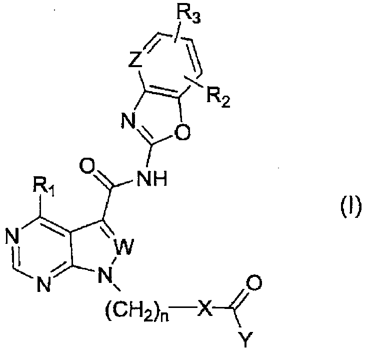

The compound represented by the above formula (I) of the present disclosure is a compound having a 1H-pyrazolo[3,4-d]pyrimidine skeleton or a 7H-pyrrolo[2,3-d]pyrimidine skeleton, wherein the compound has a benzoxazole group or an oxazolopyridine group via an amide bond. The compound represented by the above formula (I) is a BTK inhibitor having an inhibitory activity against BTK.

In the present description, examples of the "substituent" may include a halogen atom, a hydroxyl group, a cyano group, a nitro group, an alkyl group, a halogenoalkyl group, a cycloalkyl group, a cycloalkyl-alkyl group, an aralkyl group, an alkenyl group, an alkynyl group, an alkoxy group, a halogenoalkoxy group, a cycloalkoxy group, a cycloalkyl-alkoxy group, an aralkyloxy group, an alkylthio group, a cycloalkyl-alkylthio group, an amino group, a mono- or di-alkylamino group, a cycloalkyl-alkylamino group, an acyl group, an acyloxy group, an oxo group, a carboxyl group, an alkoxycarbonyl group, an aralkyloxycarbonyl group, a carbamoyl group, a saturated or unsaturated heterocyclic group, an aromatic hydrocarbon group, and a saturated heterocyclic oxy group. When the above-described substituent(s) are present, the number of the substituents is typically 1, 2, or 3.

In the present description, examples of the "halogen atom" may include a fluorine atom, a chlorine atom, a bromine atom, and an iodine atom.

In the present description, the "alkyl group" may be either a linear or branched alkyl group. Examples of the alkyl group may include C1-C6 alkyl groups, such as a methyl group, an ethyl group, an n-propyl group, an isopropyl group, an n-butyl group, an isobutyl group, a tert-butyl group, an n-pentyl group, an isopentyl group, and a hexyl group.

In the present description, the "halogenoalkyl group" is a linear or branched alkyl group having 1 to 13 halogen atoms and containing 1 to 6 carbon atoms (a halogeno C1-C6 alkyl group). Examples of the halogenoalkyl group may include halogeno C1-C6 alkyl groups, such as a fluoromethyl group, a difluoromethyl group, a trifluoromethyl group, a trichloromethyl group, a fluoroethyl group, a 1,1,1-trifluoroethyl group, a monofluoro-n-propyl group, a perfluoro-n-propyl group, and a perfluoroisopropyl group, and preferably, halogeno C1-C4 alkyl groups.

In the present description, specific examples of the "cycloalkyl group" may include C3-C7 cycloalkyl groups, such as cyclopropyl, cyclobutyl, cyclopentyl, cyclohexyl, and cycloheptyl. In the present description, the term "cycloalkylene" means divalent cycloalkyl.

In the present description, examples of the "cycloalkyl-alkyl group" may include C3-C7 cycloalkyl-substituted C1-C4 alkyl groups, such as a cyclopropylmethyl group, a cyclobutylmethyl group, a cyclopentylmethyl group, a cyclohexylmethyl group, and a cycloheptylmethyl group.

In the present description, examples of the "aralkyl group" may include C7-C13 aralkyl groups, such as a benzyl group, a phenethyl group, a naphthylmethyl group, and a fluorenylmethyl group.

In the present description, the "alkenyl group" may be any of linear, branched, and cyclic alkenyl groups, and it means an unsaturated hydrocarbon group having at least one double bond. Examples of the alkenyl group may include C2-C6 alkenyl groups, such as a vinyl group, an allyl group, a 1-propenyl group, a 2-methyl-2-propenyl group, an isopropenyl group, a 1-, 2- or 3-butenyl group, a 2-, 3- or 4-pentenyl group, a 2-methyl-2-butenyl group, a 3-methyl-2-butenyl group, a 5-hexenyl group, a 1-cyclopentenyl group, a 1-cyclohexenyl group, and a 3-methyl-3-butenyl group.

In the present description, the "alkynyl group" may be any of linear, branched, and cyclic alkynyl groups, and it means an unsaturated hydrocarbon group having at least one triple bond. Examples of the alkynyl group may include C2-C6 alkynyl groups, such as an ethynyl group, a 1- or 2-propynyl group, a 1-, 2- or 3-butynyl group, and a 1-methyl-2-propynyl group.

In the present description, the "alkoxy group" may be either a linear or branched alkoxy group. Examples of the alkoxy group may include C1-C6 alkoxy groups, such as a methoxy group, an ethoxy group, a propoxy group, an isopropoxy group, a butoxy group, an isobutoxy group, a tert-butoxy group, a pentyloxy group, an isopentyloxy group, and a hexyloxy group.

In the present description, the "halogenoalkoxy group" is a linear or branched alkoxy group having 1 to 13 halogen atoms and containing 1 to 6 carbon atoms (a halogeno C1-C6 alkoxy group). Examples of the halogenoalkoxy group may include halogeno C1-C6 alkoxy groups, such as a fluoromethoxy group, a difluoromethoxy group, a trifluoromethoxy group, a trichloromethoxy group, a fluoroethoxy group, a 1,1,1-trifluoroethoxy group, a monofluoro-n-propoxy group, a perfluoro-n-propoxy group and a perfluoro-isopropoxy group, and preferably, halogeno C1-C4 alkoxy groups.

In the present description, specific examples of the "cycloalkoxy group" may include C3-C7 cycloalkoxy groups, such as a cyclopropoxy group, a cyclobutoxy group, a cyclopentyloxy group, a cyclohexyloxy group, and a cycloheptyloxy group.

In the present description, examples of the "cycloalkyl-alkoxy group" may include C3-C7 cycloalkyl-substituted C1-C4 alkoxy groups, such as a cyclopropylmethoxy group, a cyclobutylmethoxy group, a cyclopentylmethoxy group, a cyclohexylmethoxy group, and a cycloheptylmethoxy group.

In the present description, examples of the "aralkyloxy group" may include C7-C13 aralkyloxy groups, such as a benzyloxy group, a phenethyloxy group, a naphthylmethyloxy group, and a fluorenylmethyloxy group.

In the present description, the "alkylthio group" may be either a linear or branched alkylthio group. Examples of the alkylthio group may include C1-C6 alkylthio groups, such as a methylthio group, an ethylthio group, an n-propylthio group, an isopropylthio group, an n-butylthio group, an isobutylthio group, a tert-butylthio group, an n-pentylthio group, an isopentylthio group, and a hexylthio group.

In the present description, examples of the "cycloalkyl-alkylthio group" may include C3-C7 cycloalkyl-substituted C1-C4 alkylthio groups, such as a cyclopropylmethylthio group, a cyclobutylmethylthio group, a cyclopentylmethylthio group, a cyclohexylmethylthio group, and a cycloheptylmethylthio group.

In the present description, examples of the "monoalkylamino group" may include amino groups that are monosubstituted with a linear or branched C1-C6 alkyl group, such as a methylamino group, an ethylamino group, an n-propylamino group, an isopropylamino group, an n-butylamino group, an isobutylamino group, a tert-butylamino group, an n-pentylamino group, an isopentylamino group, and a hexylamino group.

In the present description, examples of the "dialkylamino group" may include amino groups that are disubstituted with a linear or branched C1-C6 alkyl group, such as a dimethylamino group, a diethylamino group, a di(n-propyl)amino group, a diisopropylamino group, a di(n-butyl)amino group, a diisobutylamino group, a di(tert-butyl)amino group, a di(n-pentyl)amino group, a diisopentylamino group, and a dihexylamino group.

In the present description, examples of the "cycloalkyl-alkylamino group" may include C3-C7 cycloalkyl-substituted C1-C4 alkylamino groups, such as a cyclopropylmethylamino group, a cyclobutylmethylamino group, a cyclopentylmethylamino group, a cyclohexylmethylamino group, and a cycloheptylmethylamino group.

In the present description, the "acyl group" means an alkylcarbonyl group or an arylcarbonyl group.

In the present description, examples of the "alkylcarbonyl group" may include linear or branched (C1-C6 alkyl)carbonyl groups, such as methylcarbonyl, ethylcarbonyl, n-propylcarbonyl, isopropylcarbonyl, n-butylcarbonyl, isobutylcarbonyl, tert-butylcarbonyl, n-pentylcarbonyl, isopentylcarbonyl, and hexylcarbonyl.

In the present description, examples of the "arylcarbonyl group" may include (C6-C18 aryl)carbonyl groups, such as phenylcarbonyl, naphthylcarbonyl, fluorenylcarbonyl, anthrylcarbonyl, biphenylylcarbonyl, tetrahydronaphthylcarbonyl, chromanylcarbonyl, 2,3-dihydro-1,4-dioxanaphthalenylcarbonyl, indanylcarbonyl, and phenanthrylcarbonyl.

In the present description, the "acyloxy group" means an alkylcarbonyloxy group or an arylcarbonyloxy group.

In the present description, examples of the "alkylcarbonyloxy group" may include linear or branched (C1-C6 alkyl)carbonyloxy groups, such as methylcarbonyloxy, ethylcarbonyloxy, n-propylcarbonyloxy, isopropylcarbonyloxy, n-butylcarbonyloxy, isobutylcarbonyloxy, tert-butylcarbonyloxy, n-pentylcarbonyloxy, isopentylcarbonyloxy, and hexylcarbonyloxy.

In the present description, examples of the "arylcarbonyloxy group" may include (C6-C18 aryl)carbonyloxy groups, such as phenylcarbonyloxy, naphthylcarbonyloxy, fluorenylcarbonyloxy, anthrylcarbonyloxy, biphenylylcarbonyloxy, tetrahydronaphthylcarbonyloxy, chromanylcarbonyloxy, 2,3-dihydro-1,4-dioxanaphthalenylcarbonyloxy, indanylcarbonyloxy, and phenanthrylcarbonyloxy.

In the present description, the "alkoxycarbonyl group" may be either a linear or branched alkoxycarbonyl group. Examples of the alkoxycarbonyl group may include (C1-C6 alkoxy)carbonyl groups, such as a methoxycarbonyl group, an ethoxycarbonyl group, a propoxycarbonyl group, an isopropoxycarbonyl group, a butoxycarbonyl group, an isobutoxycarbonyl group, a tert-butoxycarbonyl group, a pentyloxycarbonyl group, an isopentyloxycarbonyl group, and a hexyloxycarbonyl group.

In the present description, examples of the "aralkyloxycarbonyl group" may include (C7-C13 aralkyl)oxycarbonyl groups, such as a benzyloxycarbonyl group, a phenethyloxycarbonyl group, a naphthylmethyloxycarbonyl group, and a fluorenylmethyloxycarbonyl group.

In the present description, the "saturated heterocyclic group" means a saturated heterocyclic group having a heteroatom selected from a nitrogen atom, an oxygen atom, and a sulfur atom. Specific examples of the saturated heterocyclic group may include a morpholino group, a 1-pyrrolidinyl group, a piperidino group, a piperazinyl group, a 4-methyl-1-piperazinyl group, a tetrahydrofuranyl group, a tetrahydropyranyl group, a tetrahydrothiophenyl group, a thiazolidinyl group, and an oxazolidinyl group.

In the present description, the "unsaturated heterocyclic group" means a monocyclic or polycyclic, completely unsaturated or partially unsaturated heterocyclic group. Specific examples of the unsaturated heterocyclic group may include an imidazolyl group, a thienyl group, a furyl group, a pyrrolyl group, an oxazolyl group, an isoxazolyl group, a thiazolyl group, an isothiazolyl group, a thiadiazolyl group, a pyrazolyl group, a triazolyl group, a tetrazolyl group, a pyridyl group, a pyrazyl group, a pyrimidinyl group, a pyridazinyl group, an indolyl group, an isoindolyl group, an indazolyl group, a triazolopyridyl group, a benzimidazolyl group, a benzoxazolyl group, a benzothiazolyl group, a benzothienyl group, a benzofuranyl group, a purinyl group, a quinolyl group, an isoquinolyl group, a quinazolinyl group, a quinoxalinyl group, a methylenedioxyphenyl group, an ethylenedioxyphenyl group, and a dihydrobenzofuranyl group.

In the present description, examples of the "aromatic hydrocarbon group" may include C6-C14 aromatic hydrocarbon groups, such as a phenyl group, a tolyl group, a xylyl group, a naphthyl group, an anthracenyl group, a phenanthryl group, a fluorenyl group, and a tetrahydronaphthyl group.

In the present description, the "saturated heterocyclic oxy group" means an oxy group, to which a saturated heterocyclic ring having a heteroatom selected from a nitrogen atom, an oxygen atom and a sulfur atom binds. Specific examples of the saturated heterocyclic oxy group may include a morpholinyloxy group, a 1-pyrrolidinyloxy group, a piperidinooxy group, a piperazinyloxy group, a 4-methyl-1-piperazinyloxy group, a tetrahydrofuranyloxy group, a tetrahydropyranyloxy group, a tetrahydrothiophenyloxy group, a thiazolidinyloxy group, and an oxazolidinyloxy group.

It is to be noted that, regarding the description of substituents in the present description, the expression "CA-CB" means that it is a substituent containing an A to B number of carbon atoms. For example, the term "C1-C6 alkyl group" means an alkyl group containing 1 to 6 carbon atoms, whereas the term "C6-C14 aromatic hydrocarbon oxy group" means an oxy group, to which an aromatic hydrocarbon group containing 6 to 14 carbon atoms binds. In addition, the expression "A- to B-membered" means that the number of atoms constituting a ring (the number of ring members) is A to B. For example, the term "4- to 10-membered saturated heterocyclic group" means a saturated heterocyclic group having 4 to 10 ring members.

In the general formula (I), X represents nitrogen-containing C3-C10 heterocycloalkylene optionally having a substituent. Specifically, X represents divalent heterocycloalkylene containing 3 to 10 carbon atoms, which optionally has a substituent, contains at least one nitrogen atom in the ring thereof, and further contains, in the ring thereof, 0 to 2 same or different types of heteroatoms selected from oxygen atoms or sulfur atoms (i.e. nitrogen-containing C3-C10 heterocycloalkylene). Specific examples of the nitrogen-containing C3-C10 heterocycloalkylene may include azetidinylene, pyrrolidinylene, piperidinylene, piperazinylene, morpholinylene, octahydroquinolinylene , and octahydroindolylene.

X is preferably heterocycloalkylene containing 3 to 5 carbon atoms, which optionally has a substituent and contains one nitrogen atom in the ring thereof (i.e. nitrogen-containing C3-C5 heterocycloalkylene), more preferably azetidinylene, pyrrolidinylene, or piperidinylene, and further preferably 1,3-azetidinylene, 1,3-pyrrolidinylene, or 1,3-piperidinylene.

Examples of substituents on these heterocycloalkylenes may include those as described above. However, these heterocycloalkylenes are preferably not substituted.

The nitrogen atom of the nitrogen-containing C3-C10 heterocycloalkylene group represented by X preferably binds to the carbonyl group of -COY in the general formula (I).

Furthermore, the nitrogen atom of the nitrogen-containing C3-C5 heterocycloalkylene group represented by X preferably binds to the carbonyl group of -COY in the general formula (I).

In the general formula (I), W and Z each independently represent N or CH, and preferably, Z is N and W is N, or Z is CH and W is N or CH.

In the general formula (I), n represents an integer of 0 to 2, and preferably represents 0.

In the general formula (I), examples of the "substituent" in the "amino group optionally having a substituent" represented by R₁ may include those as described above. However, the amino group optionally having a substituent represented by R₁ is preferably not substituted.

The "amino group optionally having a substituent" represented by R₁ is preferably an amino group.

In the general formula (I), R₂ and R₃, which are the same or different, each represent a hydrogen atom, a halogen atom, a C1-C6 alkyl group optionally having a substituent, a C1-C6 alkoxy group optionally having a substituent, a C3-C7 cycloalkyl group optionally having a substituent, a C6-C14 aromatic hydrocarbon group optionally having a substituent, a 4- to 10-membered monocyclic or polycyclic unsaturated heterocyclic group optionally having a substituent and containing 1 to 3 heteroatoms of the same or different types selected from a nitrogen atom, an oxygen atom and a sulfur atom, or a cyano group.

In the general formula (I), the "halogen atom" represented by R₂ or R₃ is preferably a fluorine atom, a chlorine atom, or a bromine atom.

In the general formula (I), the "C1-C6 alkyl group" in the "C1-C6 alkyl group optionally having a substituent" represented by R₂ or R₃ is preferably a C1-C4 alkyl group, more preferably a methyl group, an ethyl group, an n-propyl group, an isopropyl group, an n-butyl group, an isobutyl group, or a tert-butyl group, and further preferably a methyl group or an ethyl group.

The "substituent" in the "C1-C6 alkyl group optionally having a substituent" represented by R₂ or R₃ is preferably not substituted, or is a halogen atom or a C1-C4 alkoxy group, and is more preferably not substituted, or is a fluorine atom or a methoxy group. When the C1-C6 alkyl group optionally having a substituent has a substituent(s), the number of such substituents is not particularly limited. When the substituent is a halogen atom, the number of the substituents is preferably 1 to 3. When the substituent is a C1-C4 alkoxy group, the number of the substituents is preferably 1.

The "C1-C6 alkyl group optionally having a substituent" represented by R₂ or R₃ is preferably a C1-C6 alkyl group, a halogeno C1-C6 alkyl group, or a C1-C4 alkoxy-substituted C1-C6 alkyl group, more preferably a C1-C4 alkyl group, a halogeno C1-C4 alkyl group, or a C1-C4 alkoxy-substituted C1-C4 alkyl group, even more preferably a methyl group, an ethyl group, an n-propyl group, an isopropyl group, an n-butyl group, an isobutyl group, a tert-butyl group, a trifluoromethyl group, a trichloromethyl group, a methoxyethyl group, or an ethoxyethyl group, and further preferably a methyl group, a trifluoromethyl group, or a methoxyethyl group.

In the general formula (I), the "C1-C6 alkoxy group" in the "C1-C6 alkoxy group optionally having a substituent" represented by R₂ or R₃ is preferably a "C1-C4 alkoxy group," more preferably a methoxy group, an ethoxy group, an isopropoxy group, or an n-butoxy group, and further preferably a methoxy group.

Examples of the "substituent" in the "C1-C6 alkoxy group optionally having a substituent" represented by R₂ or R₃ may include those as described above. However, the C1-C6 alkoxy group optionally having a substituent represented by R₂ or R₃ is preferably not substituted.

The "C1-C6 alkoxy group optionally having a substituent" represented by R₂ or R₃ is preferably a C1-C6 alkoxy group, more preferably a C1-C4 alkoxy group, even more preferably a methoxy group, an ethoxy group, an isopropoxy group, or an n-butoxy group, and further preferably a methoxy group.

In the general formula (I), the "C3-C7cycloalkyl group" in the "C3-C7 cycloalkyl group optionally having a substituent" represented by R₂ or R₃ is preferably a C3-C6 cycloalkyl group, and more preferably a cyclopropyl group, a cyclobutyl group, a cyclopentyl group, or a cyclohexyl group.

Examples of the "substituent" in the "C3-C7 cycloalkyl group optionally having a substituent" represented by R₂ or R₃ may include those as described above. However, the C3-C7 cycloalkyl group optionally having a substituent represented by R₂ or R₃ is preferably not substituted.

The "C3-C7 cycloalkyl group optionally having a substituent" represented by R₂ or R₃ is preferably a C3-C6 cycloalkyl group, and more preferably a cyclopropyl group, a cyclobutyl group, a cyclopentyl group, or a cyclohexyl group.

In the general formula (I), the "C6-C14 aromatic hydrocarbon group" in the "C6-C14 aromatic hydrocarbon group optionally having a substituent" represented by R₂ or R₃ is preferably a phenyl group or a naphthyl group, and more preferably a phenyl group.

Examples of the "substituent" in the "C6-C14 aromatic hydrocarbon group optionally having a substituent" represented by R₂ or R₃ is preferably not substituted or a halogen atom, and is more preferably not substituted, or a chlorine atom or a fluorine atom. When the C6-C14 aromatic hydrocarbon group optionally having a substituent has a substituent(s), the number of such substituents is not particularly limited, and it is preferably 1 to 3.

The "C6-C14 aromatic hydrocarbon group optionally having a substituent" represented by R₂ or R₃ is preferably a phenyl group or naphthyl group that is not substituted or a phenyl group or naphthyl group that is substituted with a halogen atom, more preferably a phenyl group, a chlorophenyl group, a fluorophenyl group, a dichlorophenyl group, or a trichlorophenyl group, further preferably a phenyl group or a chlorophenyl group, and particularly preferably a phenyl group or a 4-chlorophenyl group.

In the general formula (I), the "4- to 10-membered monocyclic or polycyclic unsaturated heterocyclic group... containing 1 to 3 same or different types of heteroatoms selected from a nitrogen atom, an oxygen atom and a sulfur atom" in the "4-to 10-membered monocyclic or polycyclic unsaturated heterocyclic group optionally having a substituent and containing 1 to 3 same or different types of heteroatoms selected from a nitrogen atom, an oxygen atom and a sulfur atom" represented by R₂ or R₃ is preferably a 4- to 6-membered monocyclic unsaturated heterocyclic group containing one nitrogen atom, oxygen atom or sulfur atom, more preferably a 4- to 6-membered monocyclic unsaturated heterocyclic group containing one sulfur atom, even more preferably a thienyl group, and further preferably a 2-thienyl group.

Examples of the "substituent" in the "4- to 10-membered monocyclic or polycyclic unsaturated heterocyclic group optionally having a substituent and containing 1 to 3 same or different types of heteroatoms selected from a nitrogen atom, an oxygen atom and a sulfur atom" represented by R₂ or R₃ may include those as described above. However, this 4- to 10-membered monocyclic or polycyclic unsaturated heterocyclic group is preferably not substituted.

The "4- to 10-membered monocyclic or polycyclic unsaturated heterocyclic group optionally having a substituent and containing 1 to 3 same or different types of heteroatoms selected from a nitrogen atom, an oxygen atom and a sulfur atom" represented by R₂ or R₃ is preferably a 4- to 6-membered monocyclic unsaturated heterocyclic group containing one nitrogen atom, oxygen atom or sulfur atom, more preferably a 4- to 6-membered monocyclic unsaturated heterocyclic group containing one sulfur atom, even more preferably a thienyl group, and further preferably a 2-thienyl group.

In the general formula (I), Y is -C(R₄)=C(R₅)(R₆) or -C≡C-R₇.

In the general formula (I), R₄, R₅, R₆ and R₇, which are the same or different, each represent a hydrogen atom or a C1-C6 alkyl group optionally having a substituent.

In the general formula (I), the "C1-C6 alkyl group" in the "C1-C6 alkyl group optionally having a substituent" represented by R₄, R₅, or R₆ is preferably a C1-C4 alkyl group, more preferably a methyl group, an ethyl group, an n-propyl group, an isopropyl group, an n-butyl group, an isobutyl group, or a tert-butyl group, and further preferably a methyl group.

The "substituent" in the "C1-C6 alkyl group optionally having a substituent" represented by R₄, R₅, or R₆ is preferably not substituted, or an amino group substituted with two C1-C4 alkyl groups (wherein the C1-C4 alkyl groups may form a 4- to 8-membered ring heterocycloalkyl group together with nitrogen atoms to which these groups bind), and is more preferably not substituted, or a dimethylamino group, a methylethylamino group, a diethylamino group, a methylisopropylamino group, a 1-piperidinyl group, or a 1-pyrrolidinyl group. When the "C1-C6 alkyl group optionally having a substituent" has a substituent(s), the number of the substituents is not particularly limited, and it is preferably 1.

The "C1-C6 alkyl group optionally having a substituent" represented by R₄, R₅, or R₆ is preferably a C1-C4 alkyl group, or a C1-C4 alkyl group substituted with an amino group substituted with two C1-C4 alkyl groups (wherein the C1-C4 alkyl groups may form a 4- to 8-membered ring heterocycloalkyl group together with nitrogen atoms to which these groups bind), and more preferably a methyl group, an ethyl group, an n-propyl group, an isopropyl group, an n-butyl group, a dimethylaminomethyl group, a methylethylaminomethyl group, a diethylaminomethyl group, a methylisopropylaminomethyl group, a dimethylaminoethyl group, a diethylaminoethyl group, a 1-piperidinylmethyl group, or a 1-pyrrolidinylmethyl group.

In the general formula (I), the "C1-C6 alkyl group" in the "C1-C6 alkyl group optionally having a substituent" represented by R₇ is preferably a C1-C4 alkyl group, more preferably a methyl group, an ethyl group, an n-propyl group, an isopropyl group, or an n-butyl group, and further preferably a methyl group.

Examples of the "substituent" in the "C1-C6 alkyl group optionally having a substituent" represented by R₇ may include those as described above. However, the C1-C6 alkyl group optionally having a substituent is preferably not substituted.

The "C1-C6 alkyl group optionally having a substituent" represented by R₇ is preferably a C1-C4 alkyl group, more preferably a methyl group, an ethyl group, an n-propyl group, an isopropyl group, or an n-butyl group, and further preferably a methyl group.

In the general formula (I), the -C(R₄)=C(R₅)(R₆) or -C≡C-R₇ represented by Y is particularly preferably any one selected from the following.

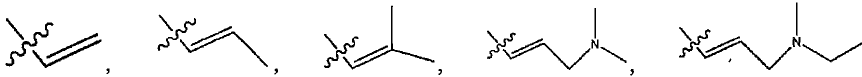

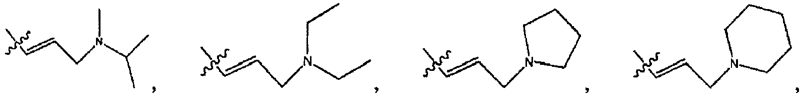

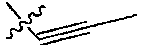

In one aspect of the present disclosure, the compound represented by the general formula (I) is preferably a compound or a salt thereof, wherein, in the general formula (1),
X is nitrogen-containing C3-C10 heterocycloalkylene;
Y is -C(R₄)=C(R₅)(R₆) or -C≡C-R₇;
W and Z are each independently N or CH;
n is 0;
R₁ is an amino group;
R₂ and R₃, which are the same or different, are a hydrogen atom, a halogen atom, a C1-C6 alkyl group optionally having a substituent, a C1-C6 alkoxy group optionally having a substituent, a C3-C7 cycloalkyl group optionally having a substituent, a C6-C14 aromatic hydrocarbon group optionally having a substituent, a 4- to 10-membered monocyclic or polycyclic unsaturated heterocyclic group optionally having a substituent and containing 1 to 3 heteroatoms of the same or different types selected from a nitrogen atom, an oxygen atom and a sulfur atom, or a cyano group; and
R₄, R₅, R₆ and R₇, which are the same or different, are a hydrogen atom or a C1-C6 alkyl group optionally having a substituent.

In this case, the compound represented by the general formula (I) is more preferably a compound or a salt thereof, wherein, in the general formula (1),
X is nitrogen-containing C3-C10 heterocycloalkylene (wherein the nitrogen atom binds to the carbonyl group of -COY in the general formula (I));
Y is -C(R₄)=C(R₅)(R₆) or -C≡C-R₇;
W and Z are each independently N or CH;
n is 0;
R₁ is an amino group;
R₂ and R₃, which are the same or different, are a hydrogen atom, a halogen atom, a C1-C6 alkyl group optionally having a substituent, a C1-C6 alkoxy group optionally having a substituent, a C3-C7 cycloalkyl group optionally having a substituent, a C6-C14 aromatic hydrocarbon group optionally having a substituent, a 4- to 10-membered monocyclic or polycyclic unsaturated heterocyclic group optionally having a substituent and containing 1 to 3 heteroatoms of the same or different types selected from a nitrogen atom, an oxygen atom and a sulfur atom, or a cyano group; and
R₄, R₅, R₆ and R₇, which are the same or different, are a hydrogen atom or a C1-C6 alkyl group optionally having a substituent.

In one aspect of the disclosure, the compound represented by the general formula (I) is preferably a compound or a salt thereof, wherein, in the general formula (1),
X is azetidinylene, pyrrolidinylene, or piperidinylene;
Y is -C(R₄)=C(R₅)(R₆) or -C≡C-R₇;
W and Z are each independently N or CH;
n is 0;
R₁ is an amino group;
R₂ and R₃, which are the same or different, are a hydrogen atom, a halogen atom, a C1-C6 alkyl group optionally having a substituent, a C1-C6 alkoxy group optionally having a substituent, a C3-C7 cycloalkyl group optionally having a substituent, a C6-C14 aromatic hydrocarbon group optionally having a substituent, a 4- to 10-membered monocyclic or polycyclic unsaturated heterocyclic group optionally having a substituent and containing 1 to 3 heteroatoms of the same or different types selected from a nitrogen atom, an oxygen atom and a sulfur atom, or a cyano group; and
R₄, R₅, R₆ and R₇, which are the same or different, are a hydrogen atom or a C1-C6 alkyl group optionally having a substituent.

In this case, the compound represented by the general formula (I) is more preferably a compound or a salt thereof, wherein, in the general formula (1),
X is azetidinylene, pyrrolidinylene, or piperidinylene (wherein the nitrogen atom binds to the carbonyl group of -COY in the general formula (I));
Y is -C(R₄)=C(R₅)(R₆) or -C≡C-R₇;
W and Z are each independently N or CH;
n is 0;
R₁ is an amino group;
R₂ and R₃, which are the same or different, are a hydrogen atom, a halogen atom, a C1-C6 alkyl group optionally having a substituent, a C1-C6 alkoxy group optionally having a substituent, a C3-C7 cycloalkyl group optionally having a substituent, a C6-C14 aromatic hydrocarbon group optionally having a substituent, a 4- to 10-membered monocyclic or polycyclic unsaturated heterocyclic group optionally having a substituent and containing 1 to 3 heteroatoms of the same or different types selected from a nitrogen atom, an oxygen atom and a sulfur atom, or a cyano group; and
R₄, R₅, R₆ and R₇, which are the same or different, are a hydrogen atom or a C1-C6 alkyl group optionally having a substituent.

In one aspect of the disclosure, the compound represented by the general formula (I) is preferably a compound or a salt thereof, wherein, in the general formula (I),
X is azetidinylene, pyrrolidinylene, or piperidinylene;
Y is -C(R₄)=C(R₅)(R₆) or -C≡C-R₇;
W and Z are each independently N or CH;
n is 0;
R₁ is an amino group;
either one of R₂ and R₃ is a hydrogen atom or a C1-C6 alkyl group, and the other is a hydrogen atom, a halogen atom, a C1-C6 alkyl group, a halogeno C1-C6 alkyl group, a C1-C4 alkoxy-substituted C1-C6 alkyl group, a C1-C6 alkoxy group, a phenyl group optionally substituted with a halogen atom, a 4- to 6-membered monocyclic unsaturated heterocyclic group containing one sulfur atom, or a cyano group;
when Y is -C(R₄)=C(R₅)(R₆),
R₄, R₅, and R₆, which are the same or different, are a hydrogen atom, a C1-C6 alkyl group, a C1-C6 alkyl group substituted with an amino group substituted with two C1-C6 alkyl groups (wherein the C1-C6 alkyl groups may form a 4- to 8-membered ring heterocycloalkyl group together with nitrogen atoms to which these groups bind); and when Y is -C≡C-R₇,
R₇ is a hydrogen atom or a C1-C6 alkyl group.

In this case, the compound represented by the general formula (I) is more preferably a compound or a salt thereof, wherein, in the general formula (I),
X is azetidinylene, pyrrolidinylene, or piperidinylene (wherein the nitrogen atom binds to the carbonyl group of -COY in the general formula (I));
Y is -C(R₄)=C(R₅)(R₆) or -C≡C-R₇;
W and Z are each independently N or CH;
n is 0;
R₁ is an amino group;
either one of R₂ and R₃ is a hydrogen atom or a C1-C6 alkyl group, and the other is a hydrogen atom, a halogen atom, a C1-C6 alkyl group, a halogeno C1-C6 alkyl group, a C1-C4 alkoxy-substituted C1-C6 alkyl group, a C1-C6 alkoxy group, a phenyl group optionally substituted with a halogen atom, a 4- to 6-membered monocyclic unsaturated heterocyclic group containing one sulfur atom, or a cyano group;
when Y is-C(R₄)=C(R₅)(R₆),
R₄, R₅, and R₆, which are the same or different, are a hydrogen atom, a C1-C6 alkyl group, a C1-C6 alkyl group, a C1-C6 alkyl group substituted with an amino group substituted with two C1-C6 alkyl groups (wherein the C1-C6 alkyl groups may form a 4- to 8-membered ring heterocycloalkyl group together with nitrogen atoms to which these groups bind); and
when Y is -C≡C-R₇,
R₇ is a hydrogen atom or a C1-C6 alkyl group.

In one aspect of the disclosure, the compound represented by the general formula (I) is preferably a compound or a salt thereof, wherein, in the general formula (I),
X is 1,3-azetidinylene, 1,3-pyrrolidinylene, or 1,3-piperidinylene; Y is -C(R₄)=C(R₅)(R₆) or -C≡C-R₇;
when Z is N, W is N, and when Z is CH, W is N or CH;
n is 0;
R₁ is an amino group;
either one of R₂ and R₃ is a hydrogen atom or a C1-C4 alkyl group, and the other is a hydrogen atom, a halogen atom, a C1-C4 alkyl group, a halogeno C1-C4 alkyl group, a C1-C4 alkoxy-substituted C1-C4 alkyl group, a C1-C4 alkoxy group, a phenyl group optionally substituted with a halogen atom, a 4- to 6-membered monocyclic unsaturated heterocyclic group containing one sulfur atom, or a cyano group;
when Y is -C(R₄)=C(R₅)(R₆),
R₄, R₅, and R₆, which are the same or different, are a hydrogen atom, a C1-C6 alkyl group, or a C1-C6 alkyl group substituted with an amino group substituted with two C1-C6 alkyl groups (wherein the C1-C6 alkyl groups may form a 4- to 8-membered ring heterocycloalkyl group together with nitrogen atoms to which these groups bind); and
when Y is -C≡C-R₇,
R₇ is a hydrogen atom or a C1-C4 alkyl group.

In this case, the compound represented by the general formula (I) is more preferably a compound or a salt thereof, wherein, in the general formula (I),
X is 1,3-azetidinylene, 1,3-pyrrolidinylene, or 1,3-piperidinylene (wherein the nitrogen atom binds to the carbonyl group of -COY in the general formula (I));
Y is -C(R₄)=C(R₅)(R₆) or -C≡C-R₇;
when Z is N, W is N, and when Z is CH, W is N or CH;
n is 0;
R₁ is an amino group;
either one of R₂ and R₃ is a hydrogen atom or a C1-C4 alkyl group, and the other is a hydrogen atom, a halogen atom, a C1-C4 alkyl group, a halogeno C1-C4 alkyl group, a C1-C4 alkoxy-substituted C1-C4 alkyl group, a C1-C4 alkoxy group, a phenyl group optionally substituted with a halogen atom, a 4- to 6-membered monocyclic unsaturated heterocyclic group containing one sulfur atom, or a cyano group;
when Y is -C(R₄)=C(R₅)(R₆),
R₄, R₅, and R₆, which are the same or different, are a hydrogen atom, a C1-C6 alkyl group, or a C1-C6 alkyl group substituted with an amino group substituted with two C1-C6 alkyl groups (wherein the C1-C6 alkyl groups may form a 4- to 8-membered ring heterocycloalkyl group together with nitrogen atoms to which these groups bind); and
when Y is -C≡C-R₇,
R₇ is a hydrogen atom or a C1-C4 alkyl group.

In one aspect of the disclosure, the compound represented by the general formula (I) is preferably a compound or a salt thereof, wherein, in the general formula (I),
X is 1,3-azetidinylene, 1,3-pyrrolidinylene, or 1,3-piperidinylene; Y is -C(R₄)=C(R₅)(R₆) or -C≡C-R₇;
when Z is N, W is N, and when Z is CH, W is N or CH;
n is 0;
R₁ is an amino group;
either one of R₂ and R₃ is a hydrogen atom or a methyl group, and the other is a hydrogen atom, a halogen atom, a methyl group, a trifluoromethyl group, a methoxyethyl group, a methoxy group, a phenyl group, a 4-chlorophenyl group, a 2-thienyl group, or a cyano group;
when Y is -C(R₄)=C(R₅)(R₆),
R₄, R₅ and R₆, which are the same or different, are a hydrogen atom, a methyl group, a dimethylaminomethyl group, a methylethylaminomethyl group, a diethylaminomethyl group, a methylisopropylaminomethyl group, a 1-piperidinylmethyl group, or a 1-pyrrolidinylmethyl group; and
when Y is -C≡C-R₇,
R₇ is a methyl group.

In this case, the compound represented by the general formula (I) is more preferably a compound or a salt thereof, wherein, in the general formula (I),
X is 1,3-azetidinylene, 1,3-pyrrolidinylene, or 1,3-piperidinylene (wherein the nitrogen atom binds to the carbonyl group of -COY in the general formula (I));
Y is -C(R₄)=C(R₅)(R₆) or -C≡C-R₇;
when Z is N, W is N, and when Z is CH, W is N or CH;
n is 0;
R₁ is an amino group;
either one of R₂ and R₃ is a hydrogen atom or a methyl group, and the other is a hydrogen atom, a halogen atom, a methyl group, a trifluoromethyl group, a methoxyethyl group, a methoxy group, a phenyl group, a 4-chlorophenyl group, a 2-thienyl group, or a cyano group;
when Y is -C(R₄)=C(R₅)(R₆),
R₄, R₅ and R₆, which are the same or different, are a hydrogen atom, a methyl group, a dimethylaminomethyl group, a methylethylaminomethyl group, a diethylaminomethyl group, a methylisopropylaminomethyl group, a 1-piperidinylmethyl group, or a 1-pyrrolidinylmethyl group; and
when Y is -C≡C-R₇,
R₇ is a methyl group.

In one aspect of the disclosure, the compound represented by the general formula (I) is preferably a compound or a salt thereof, wherein, in the general formula (I),
(1) when Z is N, and W is N,
   X is 1,3-piperidinylene, and
   Y is vinyl group,
(2) when Z is CH, and W is N,
   X is 1,3-pyrrolidinylene or 1,3-piperidinylene, and
   Y is -C(R₄)=C(R₅)(R₆) or -C≡C-(R₇),
   when Y is -C(R₄)=C(R₅)(R₆),
   R₄, R₅ and R₆, which are the same or different, are a hydrogen atom, a methyl group, a dimethylaminomethyl group, a methylethylaminomethyl group, a diethylaminomethyl group, a methylisopropylaminomethyl group, a 1-piperidinylmethyl group, or a 1-pyrrolidinylmethyl group, and
   when Y is -C≡C-(R₇),
   R₇ is a methyl group, and
(3) when Z is CH, and W is CH,
   X is 1,3-azetidinylene or 1,3-pyrrolidinylene,
   Y is -C(R₄)=C(R₅)(R₆), and
   R₄, R₅ and R₆, which are the same or different, are a hydrogen atom, a dimethylaminomethyl group, a methylethylaminomethyl group, a diethylaminomethyl group, a methylisopropylaminomethyl group, a 1-piperidinylmethyl group or a 1-pyrrolidinylmethyl group;
   n is 0;
   R₁ is an amino group; and
   either one of R₂ and R₃ is a hydrogen atom or a methyl group, and the other is a hydrogen atom, a halogen atom, a trifluoromethyl group, a methoxyethyl group, a phenyl group, a 2-thienyl group, or a cyano group.

In this case, the compound represented by the general formula (I) is more preferably a compound or a salt thereof, wherein, in the general formula (I),
(1) when Z is N, and W is N,
   X is 1,3-piperidinylene (wherein the nitrogen atom binds to the carbonyl group of -COY in the general formula (I)), and
   Y is a vinyl group,
(2) when Z is CH, and W is N,
   X is 1,3-pyrrolidinylene, or 1,3-piperidinylene (wherein the nitrogen atom binds to the carbonyl group of -COY in the general formula (I)), and
   Y is -C(R₄)=C(R₅)(R₆) or -C≡C-(R₇),
   when Y is -C(R₄)=C(R₅)(R₆),
   R₄, R₅ and R₆, which are the same or different, are a hydrogen atom, a methyl group, a dimethylaminomethyl group, a methylethylaminomethyl group, a diethylaminomethyl group, a methylisopropylaminomethyl group, a 1-piperidinylmethyl group, or a 1-pyrrolidinylmethyl group, and
   when Y is -C≡C-(R₇),
   R₇ is a methyl group, and
(3) when Z is CH, and W is CH,
   X is 1,3-azetidinylene or 1,3-pyrrolidinylene (wherein the nitrogen atom binds to the carbonyl group of -COY in the general formula (I)),
   Y is -C(R₄)=C(R₅)(R₆), and
   R₄, R₅ and R₆, which are the same or different, are a hydrogen atom, a dimethylaminomethyl group, a methylethylaminomethyl group, a diethylaminomethyl group, a methylisopropylaminomethyl group, a 1-piperidinylmethyl group, or a 1-pyrrolidinylmethyl group;
   n is 0;
   R₁ is an amino group; and
   either one of R₂ and R₃ is a hydrogen atom or a methyl group, and the other is a hydrogen atom, a halogen atom, a trifluoromethyl group, a methoxyethyl group, a phenyl group, a 2-thienyl group, or a cyano group.

In one aspect of the disclosure, the compound represented by the general formula (I) is preferably a compound or a salt thereof, wherein, in the general formula (I),
X is 1,3-piperidinylene;
Y is a vinyl group;
Z is CH;
W is N;
n is 0;
R₁ is an amino group; and
either one of R₂ and R₃ is hydrogen atom, and the other is a hydrogen atom, a halogen atom, or a cyano group.

In this case, the compound represented by the general formula (I) is more preferably a compound or a salt thereof, wherein, in the general formula (I),
X is 1,3-piperidinylene (wherein the nitrogen atom binds to the carbonyl group of -COY in the general formula (I));
Y is a vinyl group;
Z is CH;
W is N;
n is 0;
R₁ is an amino group; and
either one of R₂ and R₃ is a hydrogen atom, and the other is a hydrogen atom, a halogen atom, or a cyano group.

In one aspect of the disclosure, the compound represented by the general formula (I) is particularly preferably a compound or a salt thereof, wherein, in the general formula (I),
X is 1,3-piperidinylene;
Y is a vinyl group;
Z is CH;
W is N;
n is 0;
R₁ is an amino group; and
either one of R₂ and R₃ is a hydrogen atom, and the other is a hydrogen atom or a halogen atom.

In this case, the compound represented by the general formula (I) is particularly preferably a compound or a salt thereof, wherein, in the general formula (I),
X is 1,3-piperidinylene (wherein the nitrogen atom binds to the carbonyl group of -COY in the general formula (I));
Y is a vinyl group;
Z is CH;
W is N;
n is 0;
R₁ is an amino group; and
either one of R₂ and R₃ is a hydrogen atom, and the other is a hydrogen atom or a halogen atom.

Specific compounds may include the following compounds, but are not limited thereto:
(1) (R)-1-(1-acryloylpiperidin-3-yl)-4-amino-N-(5-chlorobenzo[d]oxazol-2-yl)-1H-pyrazolo[3,4-d]pyrimidine-3-carboxamide,
(2) (R)-1-(1-acryloylpiperidin-3-yl)-4-amino-N-(5-bromobenzo[d]oxazol-2-yl)-1H-pyrazolo[3,4-d]pyrimidine-3-carboxamide,
(3) (R)-1-(1-acryloylpiperidin-3-yl)-4-amino-N-(5-(thiophen-2-yl)benzo[d]oxazol-2-yl)-1H-pyrazolo[3,4-d]pyrimidine-3-carboxamide,
(4) (R)-4-amino-N-(5-chlorobenzo[d]oxazol-2-yl)-1-(1-methacryloylpiperidin-3-yl)-1H-pyrazolo[3,4-d]pyrimidine-3-carboxamide,
(5) (R,E)-4-amino-N-(5-chlorobenzo[d]oxazol-2-yl)-1-(1-(but-2-enoyl)piperidin-3-yl)-1H-pyrazolo[3,4-d]pyrimidine-3-carboxamide,
(6) (R)-1-(1-acryloylpiperidin-3-yl)-4-amino-N-(5-cyanobenzo[d]oxazol-2-yl)-1H-pyrazolo[3,4-d]pyrimidine-3-carboxamide,
(7) (R)-1-(1-acryloylpiperidin-3-yl)-4-amino-N-(5-methoxybenzo[d]oxazol-2-yl)-1H-pyrazolo[3,4-d]pyrimidine-3-carboxamide,
(8) (R)-1-(1-acryloylpiperidin-3-yl)-4-amino-N-(5-(2-methoxyethyl)benzo[d]oxazol-2-yl)-1H-pyrazolo[3,4-d]pyrimidine-3-carboxamide,
(9) (R)-1-(1-acryloylpiperidin-3-yl)-4-amino-N-(oxazolo[4,5-b]pyridin-2-yl)-1H-pyrazolo[3,4-d]pyrimidine-3-carboxamide),
(10) (R)-1-(1-acryloylpiperidin-3-yl)-4-amino-N-(4-methylbenzo[d]oxazol-2-yl)-1H-pyrazolo[3,4-d]pyrimidine-3-carboxamide,
(11) (R)-4-amino-N-(5-fluorobenzo[d]oxazol-2-yl)-1-(1-methacryloylpiperidin-3-yl)-1H-pyrazolo[3,4-d]pyrimidine-3-carboxamide,
(12) (R)-1-(1-acryloylpiperidin-3-yl)-4-amino-N-(5-fluorobenzo[d]oxazol-2-yl)-1H-pyrazolo[3,4-d]pyrimidine-3-carboxamide,
(13) (R)-1-(1-acryloylpiperidin-3-yl)-4-amino-N-(benzo[d]oxazol-2-yl)-1H-pyrazolo[3,4-d]pyrimidine-3-carboxamide,
(14) (R,E)-4-amino-N-(benzo[d]oxazol-2-yl)-1-(1-(but-2-enoyl)piperidin-3-yl)-1H-pyrazolo[3,4-d] pyrimidine-3-carboxamide,
(15) (R,E)-4-amino-N-(5-chlorobenzo[d]oxazol-2-yl)-1-(1-(4-(dimethylamino)but-2-enoyl)piperidin-3-yl)-1H-pyrazolo[3,4-d]pyrimidine-3-carboxamide,
(16) (R,E)-4-amino-N-(5-chlorobenzo[d]oxazol-2-yl)-1-(1-(4-(ethyl(methyl)amino)but-2-enoyl)piperidin-3-yl)-1H-pyrazolo[3,4-d]pyrimidine-3-carboxamide,
(17) (R,E)-4-amino-N-(5-chlorobenzo[d]oxazol-2-yl)-1-(1-(4-(diethylamino)but-2-enoyl)piperidin-3-yl)-1H-pyrazolo[3,4-d]pyrimidine-3-carboxamide,
(18) (R,E)-4-amino-N-(5-chlorobenzo[d]oxazol-2-yl)-1-(1-(4-(isopropyl(methyl)amino)but-2-enoyl)piperidin-3-yl)-1H-pyrazolo[3,4-d]pyrimidine-3-carboxamide,
(19) (R,E)-4-amino-N-(5-chlorobenzo[d]oxazol-2-yl)-1-(1-(4-(pyrrolidin-1-yl)but-2-enoyl)piperidin-3-yl)-1H-pyrazolo[3,4-d]pyrimidine-3-carboxamide,
(20) (R,E)-4-amino-N-(5-chlorobenzo[d]oxazol-2-yl)-1-(1-(4-(piperidin-1-yl)but-2-enoyl)piperidin-3-yl)-1H-pyrazolo[3,4-d]pyrimidine-3-carboxamide,
(21) (R,E)-4-amino-N-(5-(thiophen-2-yl)benzo[d]oxazol-2-yl)-1-(1-(4-(dimethylamino)but-2-enoyl)piperidin-3-yl)-1H-pyrazolo[3,4-d]pyrimidine-3-carboxamide,
(22) (R)-4-amino-N-(benzo[d]oxazol-2-yl)-1-(1-(but-2-inoyl)piperidin-3-yl)-1H-pyrazolo[3,4-d]pyrimidine-3-carboxamide,
(23) (R)-1-(1-acryloylpiperidin-3-yl)-4-amino-N-(5,6-dimethylbenzo[d]oxazol-2-yl)-1H-pyrazolo[3,4-d]pyrimidine-3-carboxamide,
(24) (R)-1-(1-acryloylpyrrolidin-3-yl)-4-amino-N-(5-chlorobenzo[d]oxazol-2-yl)-1H-pyrazolo[3,4-d]pyrimidine-3-carboxamide,
(25) (R,E)-4-amino-N-(5-chlorobenzo[d]oxazol-2-yl)-1-(1-(but-2-enoyl)pyrrolidin-3-yl)-1H-pyrazolo[3,4-d]pyrimidine-3-carboxamide,
(26) (R,E)-4-amino-N-(5-chlorobenzo[d]oxazol-2-yl)-1-(1-(3-methylbut-2-enoyl)pyrrolidin-3-yl)-1H-pyrazolo[3,4-d]pyrimidine-3-carboxamide,
(27) (R)-1-(1-acryloylpyrrolidin-3-yl)-4-amino-N-(benzo[d]oxazol-2-yl)-1H-pyrazolo[3,4-d]pyrimidine-3-carboxamide,
(28) (R)-1-(1-acryloylpyrrolidin-3-yl)-4-amino-N-(5-(thiophen-2-yl)benzo[d]oxazol-2-yl)-1H-pyrazolo[3,4-d]pyrimidine-3-carboxamide,
(29) (R)-1-(1-acryloylpyrrolidin-3-yl)-4-amino-N-(5-methylbenzo[d]oxazol-2-yl)-1H-pyrazolo[3,4-d]pyrimidine-3-carboxamide,
(30) (R)-1-(1-acryloylpyrrolidin-3-yl)-4-amino-N-(5-fluorobenzo[d]oxazol-2-yl)-1H-pyrazolo[3,4-d]pyrimidine-3-carboxamide,
(31) (R)-1-(1-acryloylpyrrolidin-3-yl)-4-amino-N-(5-(4-chlorophenyl)benzo[d]oxazol-2-yl)-1H-pyrazolo[3,4-d]pyrimidine-3-carboxamide,
(32) (R,E)-4-amino-N-(5-chlorobenzo[d]oxazol-2-yl)-1-(1-(4-(dimethylamino)but-2-enoyl)pyrrolidin-3-yl)-1H-pyrazolo[3,4-d]pyrimidine-3-carboxamide,
(33) (R,E)-4-amino-N-(5-chlorobenzo[d]oxazol-2-yl)-1-(1-(4-(ethyl(methyl)amino)but-2-enoyl)pyrrolidin-3-yl)-1H-pyrazolo[3,4-d]pyrimidine-3-carboxamide,
(34) (R,E)-4-amino-N-(5-chlorobenzo[d]oxazol-2-yl)-1-(1-(4-(diethylamino)but-2-enoyl)pyrrolidin-3-yl)-1H-pyrazolo[3,4-d]pyrimidine-3-carboxamide,
(35) (R,E)-4-amino-N-(5-chlorobenzo[d]oxazol-2-yl)-1-(1-(4-(isopropyl(methyl)amino)but-2-enoyl)pyrrolidin-3-yl)-1H-pyrazolo[3,4-d]pyrimidine-3-carboxamide,
(36) (R,E)-4-amino-N-(5-chlorobenzo[d]oxazol-2-yl)-1-(1-(4-(pyrrolidin-1-yl)but-2-enoyl)pyrrolidin-3-yl)-1H-pyrazolo[3,4-d]pyrimidine-3-carboxamide,
(37) (R,E)-4-amino-N-(5-chlorobenzo[d]oxazol-2-yl)-1-(1-(4-(piperidin-1-yl)but-2-enoyl)pyrrolidin-3-yl)-1H-pyrazolo[3,4-d]pyrimidine-3-carboxamide,
(38) (R)-1-(1-acryloylpyrrolidin-3-yl)-4-amino-N-(5-methoxybenzo[d]oxazol-2-yl)-1H-pyrazolo[3,4-d]pyrimidine-3-carboxamide,
(39) (R)-1-(1-acryloylpyrrolidin-3-yl)-4-amino-N-(5-cyanobenzo[d]oxazol-2-yl)-1H-pyrazolo[3,4-d]pyrimidine-3-carboxamide,
(40) (R)-1-(1-acryloylpyrrolidin-3-yl)-4-amino-N-(5-(2-methoxyethyl)benzo[d]oxazol-2-yl)-1H-pyrazolo[3,4-d]pyrimidine-3-carboxamide,
(41) (R)-1-(1-acryloylpyrrolidin-3-yl)-4-amino-N-(5-phenylbenzo[d]oxazol-2-yl)-1H-pyrazolo[3,4-d]pyrimidine-3-carboxamide,
(42) (R,E)-4-amino-N-(5-phenylbenzo[d]oxazol-2-yl)-1-(1-(4-(dimethylamino)but-2-enoyl)pyrrolidin-3-yl)-1H-pyrazolo[3,4-d]pyrimidine-3-carboxamide,
(43) (R)-1-(1-acryloylpyrrolidin-3-yl)-4-amino-N-(5-(trifluoromethyl)benzo[d]oxazol-2-yl)-1H-pyrazolo[3,4-d]pyrimidine-3-carboxamide,
(44) (R,E)-4-amino-N-(5-(trifluoromethyl)benzo[d]oxazol-2-yl)-1-(1-(4-(dimethylamino)but-2-enoyl)pyrrolidin-3-yl)-1H-pyrazolo[3,4-d]pyrimidine-3-carboxamide,
(45) 1-(1-acryloylazetidin-3-yl)-4-amino-N-(5-chlorobenzo[d]oxazol-2-yl)-1H-pyrazolo[3,4-d]pyrimidine-3-carboxamide,
(46) 7-(1-acryloylazetidin-3-yl)-4-amino-N-(5-chlorobenzo[d]oxazol-2-yl)-7H-pyrrolo[2,3-d]pyrimidine-5-carboxamide,
(47) (E)-4-amino-N-(5-chlorobenzo[d]oxazol-2-yl)-7-(1-(4-(dimethylamino)but-2-enoyl)azetidin-3-yl)7H-pyrrolo[2,3-d]pyrimidine-5-carboxamide,
(48) (R)-7-(1-acryloylpyrrolidin-3-yl)-4-amino-N-(5-chlorobenzo[d]oxazol-2-yl)-7H-pyrrolo[2,3-d]pyrimidine-5-carboxamide,
(49) (R,E)-4-amino-N-(5-chlorobenzo[d]oxazol-2-yl)-7-(1-(4-(dimethylamino)but-2-enoyl)pyrrolidin-3-yl)7H-pyrrolo[2,3-d]pyrimidine-5-carboxamide,
(50) (R,E)-4-amino-N-(5-chlorobenzo[d]oxazol-2-yl)-7-(1-(4-(ethyl(methyl)amino)but-2-enoyl)pyrrolidin-3-yl)7H-pyrrolo[2,3-d]pyrimidine-5-carboxamide,
(51) (R,E)-4-amino-N-(5-chlorobenzo[d]oxazol-2-yl)-7-(1-(4-(diethylamino)but-2-enoyl)pyrrolidin-3-yl)7H-pyrrolo[2,3-d]pyrimidine-5-carboxamide,
(52) (R,E)-4-amino-N-(5-chlorobenzo[d]oxazol-2-yl)-7-(1-(4-(isopropyl(methyl)amino)but-2-enoyl)pyrrolidin-3-yl)7H-pyrrolo[2,3-d]pyrimidine-5-carboxamide,
(53) (R,E)-4-amino-N-(5-chlorobenzo[d]oxazol-2-yl)-7-(1-(4-(pyrrolidin-1-yl)but-2-enoyl)pyrrolidin-3-yl)7H-pyrrolo[2,3-d]pyrimidine-5-carboxamide,
(54) (R,E)-4-amino-N-(5-chlorobenzo[d]oxazol-2-yl)-7-(1-(4-(piperidin-1-yl)but-2-enoyl)pyrrolidin-3-yl)7H-pyrrolo[2,3-d]pyrimidine-5-carboxamide,
(55) (R)-7-(1-acryloylpyrrolidin-3-yl)-4-amino-N-(5-phenylbenzo[d]oxazol-2-yl)-7H-pyrrolo[2,3-d]pyrimidine-5-carboxamide,
(56) (R,E)-4-amino-N-(5-phenylbenzo[d]oxazol-2-yl)-7-(1-(4-(dimethylamino)but-2-enoyl)pyrrolidin-3-yl)7H-pyrrolo[2,3-d]pyrimidine-5-carboxamide,
(57) (R,E)-4-amino-N-(5-phenylbenzo[d]oxazol-2-yl)-7-(1-(4-(ethyl(methyl)amino)but-2-enoyl)pyrrolidin-3-yl)7H-pyrrolo[2,3-d]pyrimidine-5-carboxamide,
(58) (R,E)-4-amino-N-(5-phenylbenzo[d]oxazol-2-yl)-7-(1-(4-(diethylamino)but-2-enoyl)pyrrolidin-3-yl)7H-pyrrolo[2,3-d]pyrimidine-5-carboxamide,
(59) (R,E)-4-amino-N-(5-phenylbenzo[d]oxazol-2-yl)-7-(1-(4-(isopropyl(methyl)amino)but-2-enoyl)pyrrolidin-3-yl)7H-pyrrolo[2,3-d]pyrimidine-5-carboxamide,
(60) (R,E)-4-amino-N-(5-phenylbenzo[d]oxazol-2-yl)-7-(1-(4-(pyrrolidin-1-yl)but-2-enoyl)pyrrolidin-3-yl)7H-pyrrolo[2,3-d]pyrimidine-5-carboxamide, and
(61) (R,E)-4-amino-N-(5-phenylbenzo[d]oxazol-2-yl)-7-(1-(4-(piperidin-1-yl)but-2-enoyl)pyrrolidin-3-yl)7H-pyrrolo[2,3-d]pyrimidine-5-carboxamide.

According to the present invention the therapeutic agent for use in the treatment of IgA nephropathy or purpura nephritis is (R)-1-(1-acryloylpiperidin-3-yl)-4-amino-N-(benzo[d]oxazol-2-yl)-1H-pyrazolo[3,4-d]pyrimidine-3-carboxamide (Compound 1) or a salt thereof.

Compound 1 is a BTK inhibitor having an inhibitory activity against BTK, and is a fused pyrimidine compound represented by the following general formula (II):

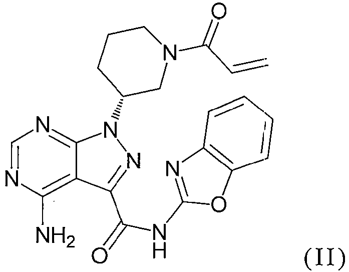

The compound represented by the formula (I) of the present invention or a salt thereof is a known compound having a BTK inhibitory activity. In the present description, the term "BTK" includes the BTK of a human or a non-human mammal, and is preferably human BTK. In addition, the term "BTK" also includes isoforms thereof.

The "BTK inhibitory activity" can be measured, for example, by the method of Test Example 1 described in International Publication WO2015/022926.

The present inventors have discovered that the compound of the present invention having a high inhibitory activity against BTK or a salt thereof is useful for the treatment of chronic glomerulonephritis (in particular, IgA nephropathy). In particular, in the case of IgA nephropathy, it has been known that IgG specific to secreted galactose-deficient sugar chain modified IgA is produced, the IgG then forms a polymer immune complex, and the IgG forming a polymer immune complex induces the production of inflammatory cytokines via FCy receptors, and that this phenomenon is deeply associated with the onset of the disease. Surprisingly, the compound of the present invention having a BTK inhibitory activity or a salt thereof has an IgG production inhibitory action (Test Example 1) and an action to inhibit the production of inflammatory cytokines via an immune complex-FCy receptor (Test Example 2), and thereby, the present compound or a salt thereof can inhibit a decrease in renal function.

According to the invention, the target disease is IgA nephropathy or purpura nephritis. In one embodiment, the target disease is IgA nephropathy.

IgA nephropathy is the most common disease among primary glomerular diseases. IgA nephropathy has findings such as glomerular hematuria and urine protein-positive urine, and is characterized by significant deposition of IgA in a mesangial region, and also, a basic disease that may be a cause of IgA nephropathy is not found. The definitive diagnosis of IgA nephropathy is carried out by pathological analysis involving, for example, PAS staining of renal biopsy samples (proliferation of mesangial cells) or a fluorescent antibody method (deposition of IgA in a mesangial region).

In the present invention, IgA nephropathy includes not only primary IgA nephropathy, but also IgA nephropathy recurred after kidney transplantation.

Purpura nephritis is nephritis considered to be one symptom of vascular purpura among chronic glomerulonephritis diseases, and it is glomerular nephritis characterized in that IgA is deposited in the glomerulus in association with purpura. The causal factor of purpura nephritis has not yet been elucidated. As with IgA nephropathy, IgA-predominant deposition is found in the renal glomerular mesangial region, and it is known that an immune complex comprising IgA is associated with this disease.

In the present invention, the term "renal function" means the function of the kidney to filter and excrete body wastes for the survival of organisms. The phrase "a decrease in renal function" means that wastes cannot be normally excreted due to the dysfunction of the kidney. Regarding methods of measuring renal function, for example, blood tests such as blood urea nitrogen (BUN), creatinine or GFR (Glomerular Filtration Rate), or urinalysis such as urine protein or urine occult blood can be carried out.

The compound of the present invention and a salt thereof can be produced according to a known organic synthesis method. For example, the compound of the present invention and a salt thereof can be produced in accordance with the methods described in International Publication WO2015/022926, International Publication WO2016/121954, etc.

When the compound of the present invention has an isomer, such as an optical isomer, a stereoisomer, a rotational isomer, or a tautomer, all of these isomers or mixtures thereof are included in the compound of the present invention, unless otherwise particularly specified. For example, when the compound of the present invention has an optical isomer, both a racemate, and an optical isomer obtained as a result of racemic resolution are included in the compound of the present invention, unless otherwise particularly specified.

The salt of the compound of the present invention means a pharmaceutically acceptable salt, and it may be, for example, a base-added salt or an acid-added salt.

The "pharmaceutically acceptable salt" means a salt having a desired pharmaceutical activity of the compound, wherein the salt is prepared from a pharmaceutical acceptable, non-toxic base or acid, including an inorganic or organic base and an inorganic or organic acid.

Specific examples of such a salt may include: acid-added salts with inorganic acids such as hydrochloric acid, hydrobromic acid, hydroiodic acid, sulfuric acid, nitric acid, and phosphoric acid; acid-added salts with organic acids such as formic acid, acetic acid, propionic acid, oxalic acid, malonic acid, succinic acid, fumaric acid, maleic acid, lactic acid, malic acid, citric acid, tartaric acid, carbonic acid, picric acid, methanesulfonic acid, paratoluenesulfonic acid, and glutamic acid; salts with inorganic bases such as sodium, potassium, magnesium, calcium, and aluminum; salts with organic bases such as methylamine, ethylamine, meglumine, and ethanolamine; salts with basic amino acids such as lysine, arginine, and ornithine; and ammonium salts. With regard to the production of such a pharmaceutically acceptable salt, the pharmaceutically acceptable salt can be synthesized, for example, according to the method described in International Publication WO2016/121954, etc.

The compound of the present invention or a salt thereof also includes a prodrug. The "prodrug" means a compound that is converted to the compound of the present invention or a salt thereof as a result of the reaction with an enzyme, stomach acid, etc. under physiological conditions in a living body; namely, a compound that enzymatically causes oxidation, reduction, hydrolysis, etc., so that it is converted to the compound of the present invention or a salt thereof, or a compound that causes hydrolysis, etc. with stomach acid or the like, so that it is converted to the compound of the present invention or a salt thereof. Otherwise, it may also be a compound that is converted to the compound of the present invention or a salt thereof under physiological conditions as described in *"*Iyakuhin no Kaihatsu (Development of Pharmaceutical Products)," Hirokawa Shoten, 1990, Vol. 7, Bunshi Sekkei (Molecular Designing), pp. 163 to 198.

The compound of the present invention or a salt thereof may be an amorphous (amorphous material) or a crystal. Although the crystal form thereof may be a single crystal or a polymorphic mixture, they are included in the compound of the present invention or a salt thereof. The crystal can be produced by crystallizing the compound of the present invention or a salt thereof, applying a known crystallization method. The compound of the present invention or a salt thereof may be either a solvate (e.g., a hydrate, etc.), or a non-solvate, and both of them are included in the compound of the present invention or a salt thereof. Compounds labeled with radioisotopes (e.g., ³H, ¹⁴C, ³⁵S, ¹²⁵I, etc.) and the like are also included in the compound of the present invention or a salt thereof. There may be a case where a plurality of crystals having spatially regular atomic arrangements and different physicochemical properties (i.e. crystal polymorphisms) are generated. The salt according to the present invention may be any of these crystal polymorphisms, and the present salt may also be a mixture of two or more crystal polymorphisms, or may further be a mixture of a crystal and an amorphous material.

In one embodiment of the present invention, the compound of the present invention or a salt thereof is a fumarate of the compound represented by the above formula (I) (the fumarate of the compound of the present invention). In a specific embodiment of the present invention, the compound of the present invention or a salt thereof is a fumarate of Compound 1. The "fumarate of the compound of the present invention" is not particularly limited, as long as it is a salt of the compound with fumaric acid, and the fumarate of the compound of the present invention includes a 1 fumarate and a 1/2 fumarate. Furthermore, the term "the fumarate of the compound of the present invention" is used to include both a crystal of the fumarate of the compound and an amorphous material of the fumarate of the compound.

In one embodiment of the present invention, the compound of the present invention or a salt thereof is a fumarate of Compound 1. The fumarate of Compound 1 is preferably a 1 fumarate of Compound 1 (which is also abbreviated as "Compound 1 - 1 fumarate") or a 1/2 fumarate of Compound 1, is more preferably a 1 fumarate of Compound 1 a (crystal), a 1/2 fumarate of Compound 1 (a crystal), or a 1 fumarate of Compound 1 (an amorphous material), and is particularly preferably a 1 fumarate of Compound 1 (a crystal) or a 1/2 fumarate of Compound 1 (a crystal).

Besides, there may be a case where a plurality of crystals having spatially regular atomic arrangements and different physicochemical properties (i.e. crystal polymorphisms) are generated. The salt according to the present invention may be any of these crystal polymorphisms, and the present salt may also be a mixture of two or more crystal polymorphisms, or may further be a mixture of a crystal and an amorphous material.

Moreover, a labeled form of the fumarate of the compound of the present invention, namely, a compound formed by substituting one or more atoms of the compound of the present invention or fumaric acid with a radioactive isotope(s) or a non-radioactive isotope(s) is also included in the present invention.

The specific form of the crystal form of the fumarate of the compound of the present invention and a method for producing the crystal form of the fumarate of the compound of the present invention are described, for example, in International Publication WO2016/121954, etc.

In the present description, the term "effective amount" used regarding the compound of the present invention means the amount of the compound of the present invention that induces the biological or medical response of a subject, such as, for example, reduction or inhibition of enzyme or protein activity; or ameliorates symptoms, alleviates conditions, and retards or delays the progression of disease; or prevents disease; etc. (therapeutically effective amount).

In the present description, the term "subject" includes mammals and non-mammals. Examples of the mammal may include, but are not limited to, a human, a chimpanzee, an ape, a monkey, a bovine, a horse, sheep, a goat, a swine, a rabbit, a dog, a cat, a rat, a mouse, a Guinea pig, a hedgehog, a kangaroo, a mole, a wild pig, a bear, a tiger, and a lion. Examples of the non-mammal may include, but are not limited to, birds, fish, and reptiles. In one embodiment, the subject is a human, and may be a human who has been diagnosed to need the treatment for the symptoms, conditions or diseases disclosed in the present description.

The age of a subject, to which the therapeutic agent of the present invention is administered, is not particularly limited. The therapeutic agent of the present invention can be used, not only to adults, but also to elderly people or children.

In the present invention the "treatment" includes, not only the treatment of the above-described chronic glomerulonephritis, in particular, the treatment of IgA nephropathy, but also includes the effect of inhibiting the progression of a decrease in renal function attended with chronic glomerulonephritis (in particular, IgA nephropathy), and alleviation of symptoms attended with chronic glomerulonephritis (in particular, IgA nephropathy).

With regard to the therapeutic agent of the present invention, only an active ingredient thereof can be provided. On the other hand, the therapeutic agent for IgA nephropathy or purpura nephritis may comprise, as necessary, a pharmaceutically acceptable carrier and the like, as well as the compound of the present invention or a salt thereof serving as an active ingredient. Thus, the therapeutic agent of the present invention can be prepared as a pharmaceutical composition consisting of one component or comprising two or more components. One embodiment of the present invention provides a pharmaceutical composition comprising the compound of the present invention or a salt thereof. The pharmaceutical composition according to one embodiment of the present invention comprises the compound of the present invention or a salt thereof, and a pharmaceutically acceptable carrier. In addition, one embodiment of the present invention provides use of the compound of the present invention or a salt thereof for producing a therapeutic agent or a pharmaceutical composition. Another embodiment of the present invention provides the compound of the present invention or a salt thereof used as a therapeutic agent or a medicine.

Another embodiment of the present invention provides Compound 1 for use in a method for treating IgA nephropathy or purpura nephritis, comprising administering an effective amount of the compound of the present invention or a salt thereof to a subject in need thereof. In one embodiment, according to this method, the production of inflammatory cytokines by IgG that forms a polymer immune complex, mediated by an immune complex-FCy receptor, is inhibited by the aforementioned administration.

Another embodiment of the present invention provides Compound 1 for use in a method for inhibiting a decrease in renal function, comprising administering the compound of the present invention or a salt thereof to a subject in need thereof. In one embodiment, the decrease in renal function is associated with IgA nephropathy.

The therapeutic agent of the present invention can be provided by the combined use with another therapeutic agent or a surgical treatment. There are no fundamental therapeutic agents for chronic glomerulonephritis (in particular, IgA nephropathy), but for example, the therapeutic agent for chronic glomerulonephritis (in particular, IgA nephropathy) can be used in combination with "tonsillectomy + steroid pulse therapy" (tonsillectomy pulse therapy), in which high dose administration (intravenous administration) of a steroid drug is combined with tonsillectomy.

The therapeutic agent of the present invention can be produced as a preparation for use in various types of administrations, as necessary, using a pharmaceutically acceptable carrier. The administration form may be either an oral administration or a parenteral administration. The form of such a preparation is not particularly limited, and examples of the preparation may include: oral agents such as a tablet, a coated tablet, a pill a powdery agent, a granule, a capsule agent, a liquid agent, a suspending agent, and an emulsion; and parenteral agents such as an injection, a suppository, and an inhalant.

One embodiment of the present invention provides a therapeutic agent for use in the treatment of IgA nephropathy or purpura nephritis for use in oral administration, comprising, as an active ingredient, the therapeutic agent of the present invention or a salt thereof. In addition, one embodiment of the present invention provides Compound 1 for use in a method for preventing and/or treating IgA nephropathy or purpura nephritis, wherein the method comprises orally administering to a subject in need thereof, an effective amount of the compound of the present invention or a salt thereof. Furthermore, one embodiment of the present invention provides the compound of the present invention or a salt thereof, which is orally administered and is used in the prevention and/or treatment of chronic glomerulonephritis.

Examples of a pharmaceutically acceptable carrier, which can be used upon molding to the form of a tablet, may include: excipients such as lactose, white sugar, sodium chloride, glucose, urea, starch, calcium carbonate, kaolin, crystalline cellulose, and silicic acid; binders such as water, ethanol, propanol, corn starch, simple syrup, glucose solution, starch solution, gelatin solution, carboxymethyl cellulose, shellac, methyl cellulose, hydroxypropyl cellulose, hydroxypropyl methyl cellulose, potassium phosphate, and polyvinyl pyrrolidone; disintegrators such as dry starch, sodium alginate, agar powder, laminaran powder, sodium hydrogen carbonate, calcium carbonate, polyoxyethylene sorbitan fatty acid esters, sodium lauryl sulfate, stearic acid monoglyceride, and lactose; disintegration inhibitors such as white sugar, stearic acid, cacao butter, and hydrogenated oil; absorption enhancers such as quaternary ammonium salt and sodium lauryl sulfate; moisturizers such as glycerin and starch; adsorbents such as starch, lactose, kaolin, bentonite, and colloidal silica; and lubricants such as purified talc, stearate, boric acid powder, and polyethylene glycol. Further, the tablet can be, as necessary, a tablet coated with an ordinary coating film, such as, for example, a sugar-coated tablet, a gelatin-coated tablet, an enteric-coated tablet, a film-coated tablet, a double-coated tablet, and a multilayered tablet.

Examples of a pharmaceutically acceptable carrier, which can be used upon molding to the form of a pill, may include: excipients such as glucose, lactose, starch, cacao butter, hydrogenated vegetable oil, kaolin, and talc; binders such as gum Arabic powder, tragacanth powder, gelatin, and ethanol; and disintegrators such as laminaran and agar. A capsule agent is prepared by mixing the compound of the present invention or a salt thereof with the above-exemplified various types of pharmaceutically acceptable carriers, and then filling the obtained mixture into a hard gelatin capsule, a soft capsule or the like according to an ordinary method.

When a liquid preparation for oral administration is produced, for example, flavoring and/or deodorant agents, a buffer agent, a stabilizer and the like are used as pharmaceutically acceptable carriers, and a liquid agent for internal use, a syrup agent, an elixir and the like can be produced. In this case, examples of the flavoring and/or deodorant agents may include white sugar, orange peel, citric acid, and tartaric acid. An example of the buffer agent may be sodium citrate. Examples of the stabilizer may include tragacanth, gum Arabic, and gelatin.

Examples of a pharmaceutically acceptable carrier, which can be used upon molding to the form of a suppository, may include polyethylene glycol, cacao butter, higher alcohol, higher alcohol esters, gelatin, and semi-synthetic glyceride.

When an injection is produced, it is preferable that a liquid agent, an emulsion and a suspending agent are sterilized and are isotonic to the blood. In addition, upon molding to such an injection, a diluent can be used as a pharmaceutically acceptable carrier. Examples of such a diluent that can be used herein may include water, lactic acid aqueous solution, ethyl alcohol, propylene glycol, macrogol, ethoxylated isostearyl alcohol, polyoxyethylenated isostearyl alcohol, and polyoxyethylene sorbitan fatty acid esters.

Besides, in this case, a common salt, glucose or glycerin in an amount sufficient for preparation of an isotonic solution may be comprised in the pharmaceutical preparation. Otherwise, an ordinary solubilizer, buffer agent, soothing agent, etc. may be added to the pharmaceutical preparation.

When an inhalant is produced, examples of the inhalant may include various types of forms such as an aerosol agent, a powdered inhalant, and a liquid inhalant.

Furthermore, each preparation as described above may comprise, as a pharmaceutically acceptable carrier, a coloring agent, a preservative, a flavoring agent, a sweetener, etc. and other pharmaceutical products, as necessary.

In the present invention, the daily dose means the amount of an active ingredient administered per day. Regarding the therapeutic agent of the present invention, the daily dose of the compound of the present invention or a salt thereof on the administration day is different depending on the symptoms, body weight, age, sex, etc. of a patient, and thus, it cannot be univocally determined. For example, the applied dose of the compound of the present invention is preferably 0.01 to 24 mg/day, more preferably 1 to 32 mg/day, and further preferably 4 to 12 mg/day, generally for an adult (body weight: 50 kg).

Moreover, the amount of Compound 1 to be mixed into each dosage unit form as described above is not constant, depending on the symptoms of a patient to whom Compound 1 is to be applied, or depending on the dosage form Compound 1, etc. In general, the amount of the dosage unit form is desirably 0.05 to 1000 mg in the case of an oral agent, it is desirably 0.01 to 500 mg in the case of an injection, and it is desirably 1 to 1000 mg in the case of a suppository.

The therapeutic agent of the present invention is determined, as appropriate, depending on various types of preparation forms, the conditions of a patient, such as age and sex, the degree of symptoms of a patient, and the like. For instance, a tablet, a pill, a powdery agent, a granule, a capsule agent, a liquid agent, a suspending agent, and an emulsion are orally administered. An injection is intravenously administered, alone or by being mixed with an ordinary fluid replacement such as glucose or amino acid, and further, as necessary, such an injection alone is administered intraarterially, intramuscularly, intradermally, subcutaneously or intraperitoneally. A suppository is intrarectally administered.

Hereinafter, the present invention will be described in more detail in the following examples.

In addition, the purposes, characteristics, advantages, and ideas of the present invention are obvious to those skilled in the art based on the present description, and thus, those skilled in the art could readily carry out the present invention based on the present description. The best mode for carrying out the present invention, specific examples, and the like show preferred embodiments of the present invention. These are described for illustrative or explanatory purposes only, and thus, are not intended to limit the scope of the present invention.

### Examples

In the following Examples, "%" indicates weight percent, unless otherwise particularly specified.

Various types of reagents used in the Examples are commercially available products, unless otherwise particularly specified.

In the following Examples, as a fumarate of Compound 1, a compound produced by the method described in International Publication WO2016/121954 was used. In addition, abbreviations have the following meanings.
DMSO: dimethyl sulfoxide
mpk: milligram/kilogram
HPMC: hypromellose

### Example 1: Effect of inhibiting IgG production in mouse B cells

The present test was carried out with reference to the Non-Patent literature (Castigli et al., J Allergy Clin Immunol 2007 120(4): 885-91). A CD43-negative B cell fraction was separated from the spleen collected from BALB/c mice, and was then suspended in a 10% FBS-containing RPMI1640 culture medium, to which an anti-CD40 antibody and IL-4 had been added. A fumarate of Compound 1 was serially diluted with DMSO, and was then added to a plate, at the same time as the seeding of the cells onto the plate. A 10% FBS-containing RPMI culture medium, to which neither an anti-CD40 antibody nor IL-4 had been added, was used as a control. DMSO, which was added to a plate at the same time as the seeding of the cells on the plate, was defined as a vehicle. The cells were cultured for 8 days at 37°C in a CO₂ incubator, and thereafter, a culture supernatant was recovered. Using an anti-IgG antibody ELISA kit (Thermo Fisher Scientific), the IgG concentration in the culture supernatant was measured.

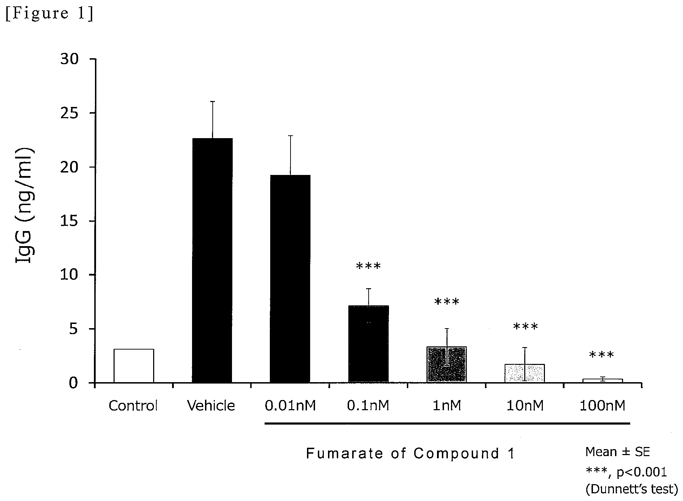

The results of the measured IgG concentration are shown in Figure 1.

It has been known that, in the case of IgA nephropathy, IgG specific to secreted galactose-deficient sugar chain modified IgA is produced, these form a polymer immune complex, and the formation of such a polymer immune complex is deeply associated with the onset of the disease.

From the results shown in Figure 1, it was found that Compound 1 inhibited IgG production in a dose-dependent manner. Since Compound 1 was confirmed to inhibit the IgG production from the B cells by stimulation with the anti-CD40 antibody and the IL-4, it was considered that Compound 1 would exhibit excellent medicinal effects on IgA nephropathy.

### Example 2: Inhibitory effects of cytokine production by IgG stimulation

The present test was carried out with reference to the Non-Patent literature (Chang et al., Arthritis Res Ther 2011 13(4): R115). Human monocytic cells THP-1 suspended in a 10% FBS-containing RPMI1640 culture medium were seeded on a 96-well plate, on which human IgG had been solid-phased. A fumarate of Compound 1 was serially diluted with DMSO, and was then added to the plate, on which the cells had been seeded. The cells seeded into a well of the plate, in which IgG had not been solid-phased, were used as a control. DMSO, which was added to the plate at the same time as the seeding of the cells, was defined as a vehicle. The cells were cultured for 4 hours at 37°C in a CO₂ incubator, and thereafter, a culture supernatant was recovered. Using an anti-TNFα ELISA kit (R & D system), the concentration of TNFα in the culture supernatant was measured.

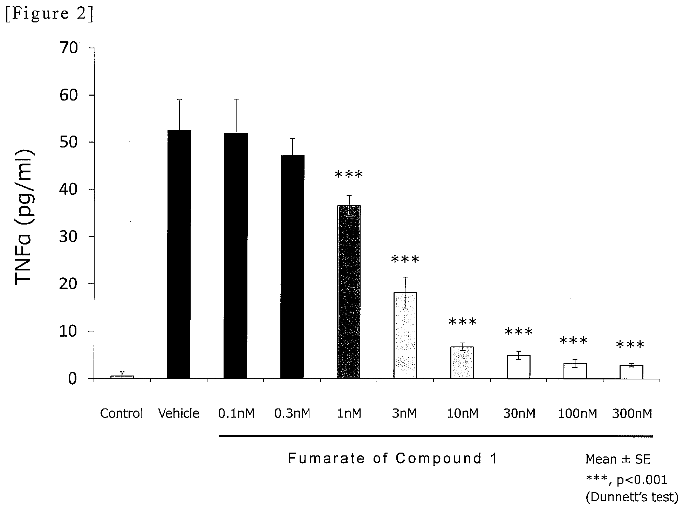

The results of the measured TNFα concentrations are shown in Figure 2.

It has been known that, in the case of IgA nephropathy, IgG that forms a polymer immune complex induces the production of inflammatory cytokines via FCy receptors, and that such induction of the production of inflammatory cytokines is deeply associated with the onset of the disease.

From the results shown in Figure 2, it was demonstrated that Compound 1 inhibited TNFα production in a dose-dependent manner. Since Compound 1 was confirmed to inhibit the TNFα cytokine production from the human monocytes by IgG stimulation, it was considered that Compound 1 would exhibit excellent medicinal effects on IgA nephropathy.

### Example 3: Inhibitory effects of renal dysfunction decrease in renal function in IgA nephropathy mouse models (HIGA mice)

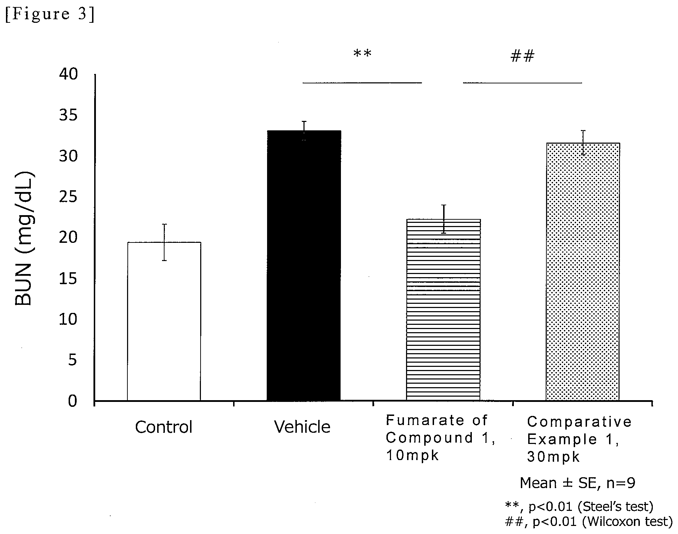

The present test was carried out, using HIGA mice that had been reported as mouse models of IgA nephropathy (Muso et al., Kidney International 1996 50: 1946-1957). A vehicle, a fumarate of Compound 1, or Ibrutinib (Comparative Example 1) was orally administered to 14-week-old to 16-week-old HIGA mice (Japan SLC, Inc.) once a day, continuously for 6 weeks. The control mice were not IgA nephropathy mouse models (HIGA mice), but were Balb/c mice (Japan SLC, Inc.) with the same age in weeks as described above. The vehicle was 0.5% HPMC. The daily doses were set as the following: the vehicle: 10 ml/kg, the fumarate of Compound 1: 10 mpk, and Ibrutinib: 30 mpk. The fumarate of Compound 1 (1 mpk) and Ibrutinib (3 mpk) are concentrations having equivalent AUC values. Thus, it is assumed that the fumarate of Compound 1 (10 mpk) and Ibrutinib (30 mpk) would have equivalent AUC values. Peripheral blood was collected from 6-week-old mice, and the urea nitrogen level in the blood was measured. Using UREA NITROGEN (BUN) Colorimetric Detection Kit (Arbor Assays), the urea nitrogen level in the blood was measured. The measured blood urea nitrogen levels (BUN) are shown in Figure 3.

It was demonstrated that there is a correlation between an increase in the blood urea nitrogen level (BUN) and a decrease in renal function (Liu et al., Renal failure 2016 38(9): 1347-1352), and a decrease in the blood urea nitrogen level (BUN) suggests the improvement of renal function.

From the results shown in Figure 3, it was demonstrated that the fumarate of Compound 1 (the present invention) inhibits an increase in the blood urea nitrogen level, when compared with the vehicle group. In addition, it was demonstrated that the fumarate of Compound 1 (the present invention) significantly reduces the blood urea nitrogen level, when compared with Comparative Example 1 (Ibrutinib).

Since Compound 1 was confirmed to significantly reduce the blood urea nitrogen level (BUN), when compared with the vehicle, it was considered that Compound 1 would exhibit excellent medicinal effects on IgA nephropathy. Moreover, even though Compound 1 was compared with Comparative Example 1 (Ibrutinib) that was a forerunner product of a BTK inhibitor, in terms of the blood urea nitrogen level (BUN), it was demonstrated that the blood urea nitrogen level was significantly reduced in the fumarate of Compound 1 administration group. Thus, the usefulness of Compound 1 as a therapeutic agent for IgA nephropathy was suggested. In the experiment using HIGA mice known as mouse models of IgA nephropathy, medicinal effects were not observed from Comparative Example 1 having the same BTK inhibitory action as that of Compound 1. In contrast, Compound 1 exhibited excellent medicinal effects. These are surprising findings.

## Claims

1. (R)-1-(1-acryloylpiperidin-3-yl)-4-amino-N-(benzo[d]oxazol-2-yl)-1H-pyrazolo[3,4-d]pyrimidine-3-carboxamide or a salt thereof for use in the treatment of IgA nephropathy or purpura nephritis.

2. A pharmaceutical composition for use in treating IgA nephropathy or purpura nephritis, which comprises, as an active ingredient, the compound defined in claim 1 or a salt thereof.

## Patentansprüche

1. (R)-1-(1-Acryloylpiperidin-3-yl)-4-amino-N-(benzo[d]oxazol-2-yl)-1H-pyrazolo[3,4-d]pyrimidin-3-carboxamid oder ein Salz davon zur Behandlung der IgA-Nephropathie oder Purpura-Nephritis.

2. Pharmazeutische Zusammensetzung zur Behandlung der IgA-Nephropathie oder Purpura-Nephritis, die als Wirkstoff die in Anspruch 1 definierte Verbindung oder ein Salz davon enthält.

## Revendications

1. (R)-1-(1-acryloylpipéridin-3-yl)-4-amino-N-(benzo[d]oxazol-2-yl)-1H-pyrazolo[3,4-d]pyrimidine-3-carboxamide ou un sel de celui-ci pour une utilisation dans le traitement de la néphropathie à IgA ou de la purpura rhumatoïde.

2. Composition pharmaceutique destinée à être utilisée dans le traitement de la néphropathie à IgA ou de la purpura rhumatoïde, qui comprend comme principe actif le composé selon la revendication 1 ou un sel de celui-ci.
